# EUROPEAN PATENT SPECIFICATION

(11) **EP 1 830 942 B1**
(45) Date of publication and mention of the grant of the patent: **20.06.2012**
(21) Application number: 05821930.4
(22) Date of filing: 22.12.2005
(51) Int. Cl.: B01D 29/60

(54) **FILTERING DEVICE COMPRISING MEANS FOR DETECTING CHARACTERISTICS OF A FLUID IN MOTOR VEHICLES**
FILTERVORRICHTUNG MIT MITTELN ZUR ERFASSUNG VON EIGENSCHAFTEN EINES FLUIDS IN KRAFTFAHRZEUGEN
DISPOSITIF DE FILTRATION AVEC DES MOYENS POUR DETECTER LES CARACTERISTIQUES D'UN FLUIDE DANS DES VEHICULES A MOTEUR

(30) Priority: 24.12.2004 IT TO20040903
(43) Date of publication of application: 12.09.2007
(73) Proprietor: ELTEK S.p.A., I-15033 Casale Monferrato (Alessandria) (IT)
(72) Inventor: GADINI, Costanzo, I-15040 Frassineto Po (Alessandria) (IT); COLOMBO, Paolo, I-15100 Alessandria (IT)
(74) Representative: Gallarotti, Franco
(86) International application number: PCT/IB2005/003960
(87) International publication number: WO 2006/067618

(56) References cited:
- EP-A- 1 462 775
- EP-A- 1 484 097
- WO-A-2005/040788
- DE-A1- 10 202 030
- US-A- 5 858 224
- US-A1- 2003 046 985
- US-A1- 2004 231 402

## Description

The present invention relates to a for detecting characteristics of a fluid, in particular of the type used on motor vehicles, said device being of particularly advantageous, albeit not exclusive, use for detecting characteristics of a lubricating fluid.

The correct operation of a lubrication circuit, for example, of an internal-combustion engine of a motor vehicle, presupposes that the oil used will maintain a good quality. The characteristics of the oil tend, however, to degrade over time, on account of contamination of various nature; for example, in the specific case of internal-combustion engines, fumes, unburnt particles, metallic residue, and fuel can occasionally come into contact with the lubricating oil of the engine, so reducing its operating characteristics.

For the purpose of preserving the quality of the oil there are thus provided purposely designed filters, operative for withholding the aforesaid contaminant elements. Notwithstanding the presence of a filter, it may, however, happen that the characteristics of the oil undergo degradation before the expiration normally recommended for its replacement, and it is clear that the premature decay of the quality of the oil can be the cause of serious damage to the lubrication system or to the engine. On the other hand, it is also possible that, at the moment of programmed replacement, the oil will still conserve excellent characteristics, which would render replacement thereof momentarily superfluous.

For the aforesaid reasons, suitably designed detection devices have been proposed, aimed at monitoring one or more qualitative characteristics of the lubricating oil. When said devices detect the decay of a certain characteristic with respect to a pre-determined threshold value, a suitable control system sees to signalling the fault in order to indicate the need to proceed to replacement of the oil.

The above devices usually comprise sensors equipped with electrodes designed for contact with the fluid or oil, between which there is, for example, applied and/or detected a difference of potential, a voltage or a signal, for the purposes of detection and/or measurement of quantities such as acidity, conductivity, resistivity, or capacitance of the oil, from which quantities it is possible to deduce some characteristics of interest regarding the oil itself, as well as its level.

In certain cases, the detection device is designed for being positioned in a specific installation seat, which necessarily implies a specific pre-arrangement of the engine of the vehicle, which must be equipped with the aforesaid seat, designed for enabling housing and hydraulic connection of the device. On account of the different structures or conformations of the engines of vehicles, the aforementioned devices and the respective seats are typically shaped so as to enable their housing in areas available for said purpose in the engine compartment, said areas being frequently difficult to access for the purposes of maintenance, for example, for periodic cleaning.

Known from the document No. US2003/0179002 is a device comprising means for detecting a condition of the oil, which are connected to a respective circuit of analysis. The detecting means, constituted by a sensor of capacitance or impedance, are integrated in the filter cartridge of an oil filter, the latter being housed in a casing formed in part by a removable component and in part by a fixed seat of the oil circuit, said fixed seat being substantially made in a part of the engine of a vehicle. In a possible embodiment, the aforesaid circuit of analysis is housed in a respective casing fixed or integrated on the outside of the aforesaid fixed seat and is connected to the detecting means, via a suitably provided line, by means of a separable connector. The solution described in the aforesaid prior document is such that the periodic replacement of the filter cartridge, for example, when occluded or clogged, determines also the replacement of the detecting means, with consequent high operating costs of the system.

US 5 858 224 A discloses of the features of the preamble of claim 1.

In its general terms, the present invention proposes solving one or more of the aforesaid drawbacks; and in particular of providing a filtering device comprising means for detecting characteristics of a fluid, said device being readily insertable or replaceable in the hydraulic system of a vehicle, without this entailing particular difficulties of assembly/disassembly and/or of operation of the system itself, which is built in such a way that it is inexpensive and does not involve high operating costs.

The above and other purposes still, which will clearly emerge from what follows, are achieved according to the present invention by a filtering device comprising means for detecting characteristics of a fluid, in particular lubricating oil used on vehicles in combination with a filter element, said device having the characteristics indicated in the annexed claims, which are understood as forming an integral part of the present description.

Further purposes, characteristics and advantages of the present invention will clearly emerge from the ensuing detailed description and from the annexed drawings, which are provided purely by way of explanatory and non-limiting example and in which:
- Figure 1 is a schematic plan view of a device not according to the invention;
- Figure 2 is a schematic cross-sectional view according to line II-II of Figure 1, with the device in a possible condition of use;
- Figure 3 is an exploded view of the device of figure 1;
- Figure 4 is a schematic cross-sectional according to a line homologous to that designated by IV-IV in Figure 2, aimed at illustrating sensor means of the device according to the invention;
- Figure 5 is an exploded view of two details of Figure 4;
- Figure 6 is a schematic cross-sectional view of a first device according to the invention;
- Figure 7 is a schematic partially exploded cross-sectional view of some details of the device illustrated in Figure 6;
- Figure 8 is a schematic cross-sectional view of a second device according to the invention;
- Figure 9 is a schematic cross-sectional view of a detail of the device illustrated in Figure 8;
- Figure 10 is a schematic cross-sectional view of a variant of the device according to the invention;
- Figures 11 is a schematic cross-sectional view of two details of the device illustrated in Figure 10;
- Figure 12 is a schematic cross-sectional view of a further variant of the device according to the invention;
- Figure 13 is a schematic cross-sectional view of a further variant of the device according to the invention;
- Figures 14 and 15 are schematic cross-sectional views of some details of the device illustrated in Figure 13;
- Figure 16 is a schematic cross-sectional view of a further variant of the device according to the invention;
- Figures 17 and 18 are schematic cross-sectional views of two details of the device illustrated in Figure 16;
- Figure 19 is a schematic cross-sectional view of a further variant of the device according to the invention;
- Figure 20 is a partial schematic cross-sectional view of a detail of the device illustrated in Figure 19;
- Figure 21 is a schematic cross-sectional view of a part of a device built in accordance with a further variant of the invention;
- Figure 22 is a schematic view, similar to the one illustrated in Figure 5, of two electrodes of a sensor means built in accordance with a further variant of the invention;
- Figure 23 is a partial and schematic cross-sectional view of a further variant of the device according to the invention; and
- Figure 24 is a schematic cross-sectional view according to the line XXVI-XXVI of Figure 23.

The idea underlying the present invention is to provide a filtering device of the type previously referred to, the active part of which, i.e., the one equipped with sensor means, is substantially integrated in a removable part of the outer casing of a filter for a fluid circulating in a vehicle, said outer casing being made up of a number of parts and defining a housing for a filter element which can be periodically replaced. In this way, the replacement of the filter element can occur independently of the possible replacement of the aforesaid removable part of the outer casing, or, independently of the possible replacement of the aforesaid sensor means associated to the removable part.

The aforesaid filter can be, for example, a cartridge filter provided for purification of the lubricating oil of an internal-combustion engine, which in use is normally connected mechanically and hydraulically to a part of the engine, in particular via a hydraulic joint comprising an inlet pipe and an outlet pipe, provided, respectively, to cause the oil that is to be filtered to flow towards the filter and then the filtered oil to flow away towards the engine.

The structure of the device according to the invention is preferably such that at least its detection part is integrated in the part of the casing of the filter, or lid, which is normally removed during the step of replacement of the filter cartridge. Alternatively, the part for detecting of the device according to the invention can be integrated in another removable portion of the casing of the filter, which in use is coupled in a separable way to the engine or to the vehicle, in particular via the aforesaid hydraulic joint.

Since it is integrated in at least one removable part of the outer casing of a filter, the device according to the invention is thus distinct and separable both from the filter element or cartridge of the filter itself, and from other parts of the casing or of the engine. This enables the part of casing integrating the device to be replaced in cartridge filters originally without sensor means. In other words, then, the device according to the invention can be mounted at a later date on a vehicle or on an engine to which only a traditional filter is originally associated, as commonly envisaged in all vehicles.

The device according to the invention will be preferably equipped with a respective control circuit, in particular of an electronic and independent type, i.e., dedicated to the device itself. In this case, the electronic control circuit can be integrated directly in the device forming the subject of the present invention. The control circuit of the device could, on the other hand, be integrated, in all or in part, in a control unit already present on the vehicle, equipped with means of interfacing and/or of interconnection to the electrical and/or electronic part of the device itself. The operating program of said unit can, if need be, be of an updateable or re-programmable type, in particular for enabling control of the device according to the invention.

The proposed solution enables the device to be readily accessed, in so far as it is located in an area subject to periodic maintenance. Consequently, the device can be conveniently replaced, if need be, or else momentarily dismantled for the purposes of cleaning or washing, which can occur during the normal operations of periodic replacement of the cartridge of the filter.

The proposed solution thus enables the filter cartridge to be replaced without having to replace the sensor, or else replacement of both of them, or else again replacement or cleaning of the part of device that integrates the sensor means, without replacing the filter.

The device is designed for detecting the quality of the fluid that that has already been filtered. Advantageously, the device according to the invention can integrate also further sensor means, such as a pressure sensor, aimed at monitoring the state of operation and/or efficiency of the filter, for example, for detecting its possible conditions of clogging, and/or a temperature sensor, for example, for correlating the measurements to the variations in temperature of the fluid and/or of the environment.

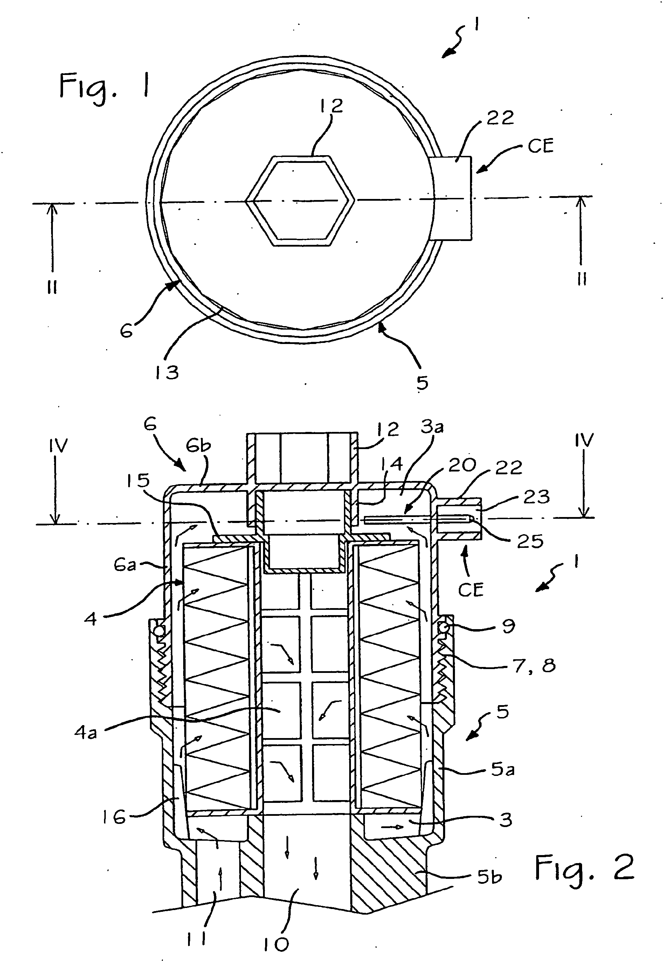

Represented in Figures 1 to 5 is a possible embodiment of a device not according to the invention. Elements disclosed in connection with the device of figures 1-5, such as the configuration of its sensor means, can be used in the device according to the invention, as described hereinafter.

In said figures, the reference number 1 designates as a whole a cartridge filter, comprising a main body or casing made up of a number of parts, defining within it a housing chamber 3 for a filter element, or cartridge, designated by 4 and having a structure in itself known, or in any case suitable for the purpose.

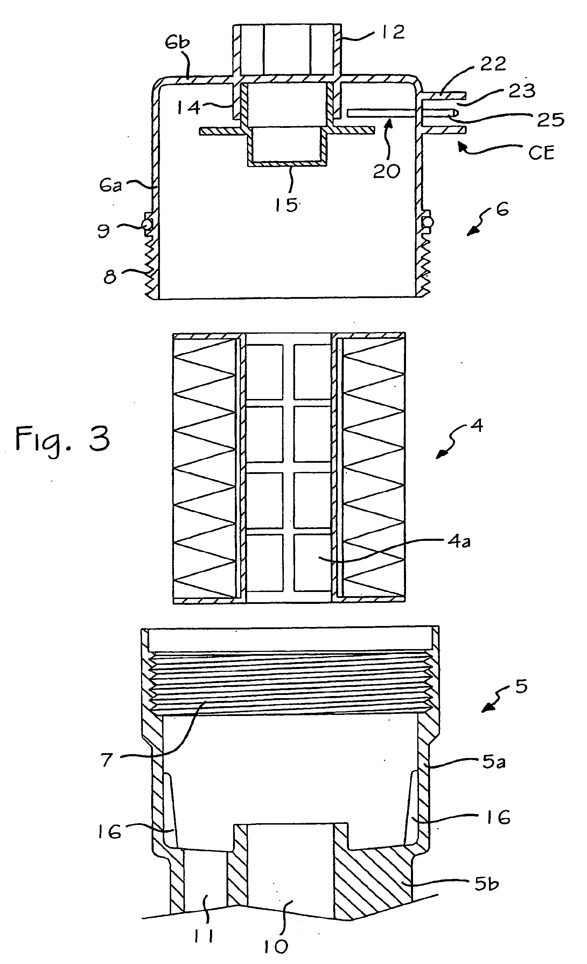

As may be seen in Figure 3, in the case exemplified the casing of the device is formed by a bottom part, referred to hereinafter as base, and a top part, referred to hereinafter as lid, designated, respectively, by 5 and 6. The two parts 5 and 6, which are generally cup-shaped, are coupled to one another in a separable way in order to enable access to the filter cartridge 4, for example, for its replacement. In the case exemplified, the coupling between the base 5 and the lid 6 is of a threaded type and comprises a female thread 7, formed on the internal surface of a cylindrical wall 5a of the base 5, and a thread 8 formed on the outer surface of a cylindrical wall 6a of the lid 6. Provided between the parts 5 and 6 are suitable sealing means 9, constituted, for example, by at least one O-ring housed in a respective seat formed in the lid 3, at the top end of the respective thread 8. Obviously, other mechanical systems are possible for joining the base 5 and the lid 6 to one another in a separable, hydraulically sealed way, such as fast couplings of the press-block type, bayonet couplings, snap-action couplings, etc. The casing 5-6 could also be made up of a larger number of parts with respect to the case illustrated (for example, with an intermediate tubular element between base and lid), and then the areas of sealed joining could be different in number or larger and/or located in different areas.

The base 5, for example made of metal material, is designed to be rendered fixed or form an integral part, in a suitably envisaged point, of a hydraulic circuit of the vehicle, for example, on the engine, possibly in a position corresponding to a respective hydraulic joint of a type in itself known.

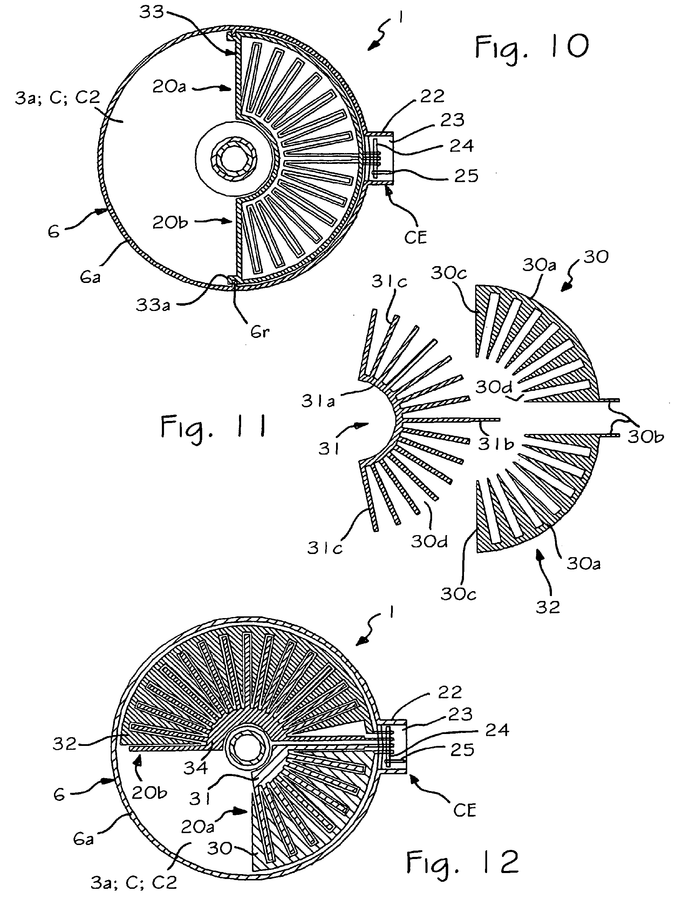

In this connection, it may be noted that the non-limiting examples of Figures 1-9 refer to a case where the base forms an integral part of an engine, or is in any case fixed in a not readily removable way with respect to the latter. In the case of the variant illustrated in Figures 10-11, instead, the aforesaid base is of a readily removable type with respect to the engine, in so far as it is mounted on a corresponding seat provided in the latter. Irrespective of the two embodiments, the end wall of the base 5, designated by 5b, has at least one central passage 10 and at least one side passage 11, or an outlet conduit and an inlet conduit for the fluid, the functions of which will clearly emerge from what follows.

The lid 6, for example made of thermoplastic material, has an end wall 6b, from which there rises at the top a first tubular portion in relief 12, shaped for receiving a tool necessary for screwing/unscrewing the lid itself with respect to the base 5. In the case exemplified, the portion 12 has a hexagonal shape, both internally and externally, in order to receive an appropriate spanner in a position corresponding to its internal or external profile. Also the outer surface of the cylindrical wall of the lid 6, in the area not concerned by the thread 8 and by the seat for the gasket 9, is provided with a polygonal profile, designated by 13 in Figure 1, i.e., with a series of flat and/or pointed surfaces or surfaces in relief in order to enable appropriate engagement or friction with purposely designed tools or else with tools already known in the sector, for example in order to fix the casing 5-6 on the respective part of engine.

Branching off from the internal part of the wall 6a of the lid 6 is a seat 14, in particular constituted by at least one respective wall or element in relief, for fixing and/or positioning of a spacer, designated by 15. The spacer 15 has the function of co-operating with the cartridge 4 in order to guarantee certain positioning and/or hydraulic sealing of the latter within the housing chamber 3 and thus correct circulation of the fluid that is to be purified, which is here assumed as being lubricating oil. The correct positioning of the cartridge 4 is likewise guaranteed by the presence of centring ribs 16 projecting from the internal surface of the base 5.

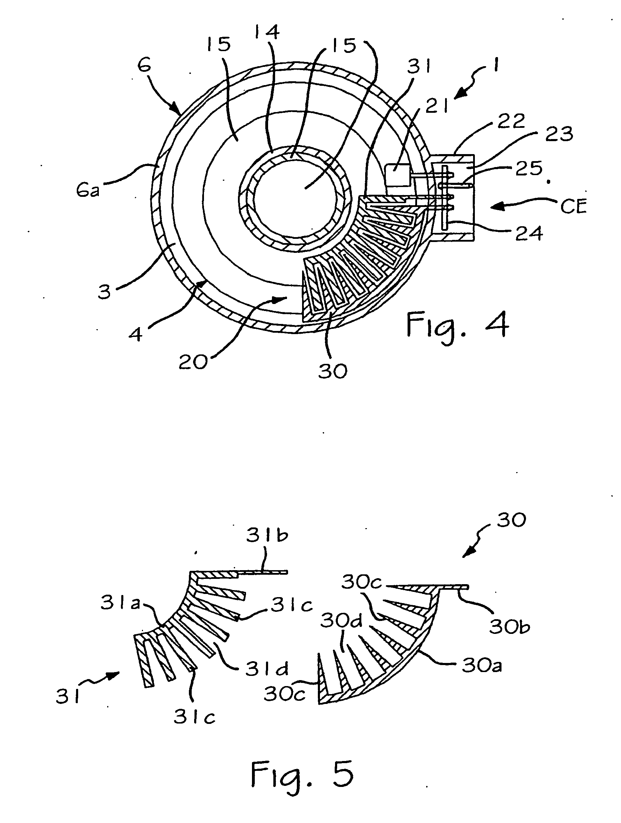

Sensor means are associated to the lid 6, for detecting one or more characteristics of the fluid or oil that is to flow through the filter 1. For said purpose, sensor means are housed in the upper region 3a of the chamber 3, which, in the case exemplified, comprise the electrodes of a sensor 20 for detecting one or more electrical quantities, for example, conductivity, resistivity, capacitance, impedance, etc. Preferably, at least one further sensor, designated by 21 in Figure 4, such as, for example, a temperature sensor and/or a quartz oscillator, is associated to the sensor 20.

The body of the lid 6 is preferably shaped so as to provide at least part of an electrical connector CE. For this purpose, the body of the lid 6 comprises a second tubular portion 22, which herein extends in a radial direction from the peripheral wall 6a, forming a cavity or hollow 23. The tubular portion 22 is preferably provided with appropriate engagement means, in particular for a complementary connector (not represented) forming part of an external wiring, to which the device 1 is to be connected. In a variant (not represented), the tubular portion 22 could be configured as an element independent of the casing 5-6, designed to be fixed to the latter, for example, via couplings, welding, bonding, overmoulding, or any other known technique.

As may be seen in Figure 4, the connection terminals of the sensor means 20, 21 are inserted through respective passages formed in the peripheral wall 6a of the lid, in a position corresponding to the compartment 23, with the respective ends that project into the compartment itself. Figure 4 further highlights how, in a possible variant embodiment, within the hollow 23 the aforesaid terminals can be electrically and mechanically connected to an electrical-connection support, designated by 24, comprising, for example, a printed circuit. Said printed circuit 24 can form part of an electronic circuit for measurement and/or control of the device 1, or of a circuit of interconnection of the aforesaid sensor means.

In order to guarantee the necessary tightness, said passages and/or said electronic circuit are appropriately sealed, for example via suitable resins or glues and/or other coating or covering elements. Alternatively, the lid 6 could be made by overmoulding the plastic material directly on at least part of said elements, or at least on the electrical terminals of the sensor means 20, 21, or on other electrical terminals to which said sensor means are subsequently connected in the course of production of the lid 6. The electrical terminals of the sensor means 20, 21 could moreover be provided with respective coating and/or protection means, i.e., be insulated, for example, against possible phenomena of corrosion due to contact with the fluid to be detected, or for the case where the part of casing 5, 6 in which they are integrated is made of metal.

To said terminals and/or to the support 24 there can be connected electrical connection elements, as designated as a whole by 25 in Figure 4, which form, together with the tubular portion 22, the electrical connector CE, for example, of a male type, suitable for coupling with a complementary connector, for example, of a female type (not represented) and in turn connected to a respective wiring and/or to a control unit of the vehicle. Preferably sealing means are provided between the aforesaid connectors, in order to prevent any infiltration of moisture and dirt into the hollow 23.

As has been mentioned, the presence of the support or circuit 24 is to be considered optional. In the case of its absence, the connection elements 25 can be obtained directly from the terminals of the sensors 20 and/or 21, or else rendered fixed to these, as exemplified, for instance, in Figures 2 and 3.

The sensor 21 can be a temperature sensor, constituted, for example, by a negative-temperature-coefficient (NTC) resistor. The variable detected by the sensor 21, in addition to being of importance in absolute terms (i.e., as knowledge of the temperature of the fluid), can also be used for the purpose of compensating the detections made using other sensor means, and this in view of the fact that some physical characteristics of a fluid can vary at its different temperatures (for example, the value of viscosity of the oil at a first temperature thereof is different from the one that can be detected at a different operating temperature of the same oil). In such a perspective, for example, in the case where the quantities detected via the means 20 have to be compared with fixed-reference data or tables, the control logic that supervises operation of the device according to the invention will see to compensating said quantities detected as a function of the temperature of the fluid existing at the moment of detection of the quantity of interest, as measured by the sensor 21. Alternatively, the control logic may envisage that given phases of detection via the means 20 will be taken when a pre-determined temperature is reached by the fluid, this point being detected by the sensor 21.

The sensor 21 could also be a generator of oscillations, constituted by a quartz crystal, used in order to measure the viscosity of the fluid, according to modalities in themselves known, and on the basis of the fact that the quartz crystal varies its own frequency of oscillation as a function of the viscosity of the fluid with which it is in contact or in which it is immersed. Given that the viscosity depends to a certain extent upon the temperature of the fluid, in a preferred embodiment of the invention, there may be provided both a temperature sensor and a viscosity sensor, with the first positioned as close as possible to the second and/or to the sensor 20 in order to have information as correct as possible as regards the viscosity and/or the other characteristics of the fluid. It is in any case evident that the sensor means, here designated for practical reasons by 20 and/or 21, may be in a number and/or of a type different and/or larger with respect to the ones represented merely by way of non-limiting example.

It should be noted that the electronic components referred to generically as "quartz components" usually comprise a casing for protection of a respective quartz crystal, connected to two electrical contacts, which when subjected to an appropriate voltage oscillates at a given frequency. In the case of the present invention, the viscosity sensor is preferably without the aforesaid casing and thus formed merely by the quartz crystal, with respective connection terminals projecting in the hollow 23. This enables a further increase in the precision of measurement made via the quartz crystal. Advantageously a perforated casing could be provided for the quartz oscillator or for other sensor means, for example, with a shape similar to a mesh, said particular type of casing being designed to protect the quartz oscillator or other sensors during handling, for example, in the production stage, and/or from accidental impact, albeit enabling the quartz oscillator itself or other sensors to come into contact with the fluid to be detected, Said perforated-casing protection could advantageously be constituted by, or associated to, at least one component of the device 1, appropriately shaped and preferably made of thermoplastic material, such as, for example, the lid 6, or supporting parts of the sensor means, etc.

The sensor 20 is, instead, provided for detecting an electrical quantity that varies as a function of the quality of the fluid, such as a value of resistance, resistivity, capacitance, acidity, etc.

A first possible embodiment of the sensor 20 is visible in the cross-sectional view of Figure 4, limitedly to its two metal parts, constituted by two electrodes 30 and 31. In Figure 5 said electrodes 30 and 31 are visible individually, as obtained starting from at least one metal strap of small thickness, appropriately blanked and shaped. It may be noted that the sensor 20 is preferably equipped also with a frame or support made of electrically insulating material, such as a thermoplastic material, not visible in the cross-sectional views of Figures 4 and 5, provided for supporting and holding the two electrodes 30 and 31 in respective fixed positions and/or for facilitating their installation and/or their housing with respect to the lid 6.

Each electrode 30, 31 comprises a respective base part 30a, 31a, substantially configured like the arc of a circle, from which there branches off a connection projection configured like a terminal 30b, 31b, in particular having a flattened shape. The terminals 30b, 31b preferably have a thickness equal or close to that of said strap from which the electrodes 30 and 31 have been obtained, or else equal to a multiple of said thickness, if the terminals are obtained by bending a portion of the strap back on itself. As may be noted, the development in length of the curved or arched base part 30a of the electrode 30 is greater than that of the homologous part 31a of the electrode 31 (in other words, the arc of a circle defined by the part 30a is longer than that defined by the part 31a). In the case exemplified, the shape that is at least partially curved or arched of the electrodes 30, 31 is advantageous in so far as it enables complete exploitation of the morphology of circular cross section of the part of the casing 5-6 in which the electrodes themselves are located.

As may be appreciated from Figure 4, the terminal projection 30b, 31b are inserted in a sealed way in respective passages formed in the side wall 6a of the lid 6 so as to project within the hollow 23 and be connected therein to the connection support 24. In a variant (not represented), the terminals 30b and 31b and/or the terminals of the further sensor means (for temperature, viscosity, etc.) could form directly the terminals 25 of the connector CE, as represented in some of the attached figures.

From the base part 30a of the electrode 30 there branches off laterally a series of first prolongations or teeth 30c having a substantially triangular or pointed shape, i.e., one that narrows towards the outer end, arranged so that the teeth themselves are spaced apart with respect to one another by spaces 30d. Said spaces 30d are delimited longitudinally by the facing surfaces of two successive teeth 30c. Said facing surfaces substantially coincide with the thickness of the strap in said area. Given the "arched" conformation of the electrode 30 and the shape of the teeth 30c, in the example of Figure 5 said facing surfaces are substantially parallel, even though other arrangements are possible (the facing surfaces could, for example, be convergent towards the outside or towards the inside, i.e., with narrower or wider spaces 30d, respectively, and widen towards the pointed ends). In the embodiment illustrated in Figure 4, the teeth 30c and/or the respective pointed ends face the centre of the device 1 or of its lid 6.

Also from the base part 31 a of the electrode 31 there branch off laterally a series of second prolongations or teeth 31c, having a substantially rectangular shape, arranged so that the teeth themselves are spaced apart with respect to one another by spaces 31 d; also said spaces 31d are delimited longitudinally by the facing surfaces of two successive teeth 31c. Said facing surfaces substantially coincide with the thickness of said strap in said area. In this case, given the arched conformation of the electrode and the shape of the teeth 31 c, said facing surfaces are substantially divergent from one another. The teeth 31 c could, however, have a different shape, for example, be substantially pointed or triangular. In addition, the respective facing surfaces could be more or differently inclined with respect to one another, for example, with spaces 31 d still wider towards the outer part. In the example illustrated in Figure 4, the teeth 31c and/or the respective pointed ends face the peripheral part of the device 1 or of its lid 6, with respect to the tangents of which they are substantially orthogonal.

As may be appreciated, the arc of a circle defined by the base part 30a of the electrode 30 has a development in length greater than an arc of an imaginary circle touched by the ends or tips of the respective teeth 30c. Instead, the arc of a circle defined by the base part 31 a of the electrode 31 has a development in length smaller than an arc of an imaginary circle touched by the ends or tips of the respective teeth 31c.

The conformation of the teeth 30c, 31c is such that, when the two electrodes 30, 31 are coplanar, in an operating position, the teeth 30c are intercalated or interdigitated to the teeth 31c, without ever coming into contact therewith, as may be seen, for example, in Figure 4, with the facing surfaces of the teeth 30c and 31 c substantially parallel to or equidistant from one another. The set of the two electrodes 30, 31 with interdigitated teeth has the overall shape of a circular sector with a development of approximately 90°, in the case represented in Figure 4.

As has been said, the electrodes 30 and 31 can, for example, each be made of a single piece from a strap, and hence in the form of a lamina having two parallel main faces or faces with larger area, between which said thickness of the lamina or strap itself is defined. In the case where the sensor 20 is of a capacitive type, the surfaces corresponding to said thickness, i.e., the perimetral surfaces obtained in the thickness of the metal strap of the two electrodes form the plate surfaces (armatures) of the sensor 20. In a variant (not represented), on the other hand the plate surfaces of the sensor 20 could be at least in part made of two main faces, or faces of larger area, set facing one another, off two respective electrodes.

In the use of the sensor 20, when the electrodes 30, 31 are immersed in the fluid and an appropriate voltage and/or current is applied and/or detected across them, possible variations of an electrical quantity (conductivity, resistivity, capacitance, acidity) measured via the electrodes themselves can be evaluated electronically, in a known way, in order to have information regarding the level of degradation of the fluid (in this regard, it should be considered, for example, that the conductivity of the oil increases with its ageing).

Obviously, the sensor means provided for detecting the electrical quantity or quantities of interest mentioned above could be built in a different way from those previously exemplified; for instance, they may be of a type commonly available on the market.

In Figure 2 the device 1 is illustrated in a condition of possible use, i.e., mounted on the engine of a vehicle. As has been said, in said version the base 5 forms an integral part of an engine or is in any case fixed in such a way that it is not readily removable from said engine. In said configuration, then, the base 5 forms a sort of bottom seat for the cartridge 4, said seat being equipped with a joint (herein constituted by the thread 8) for the lid 6 that integrates the sensor means 20, 21. The aforesaid seat comprises, and/or is in communication with, a first conduit for outlet of the oil to be filtered from the engine and a second conduit for return of the filtered oil to the engine, which, in this configuration, are at least in part formed, respectively, by the passage 11 and by the passage 10 of the base 5. Once again in Figure 2, following upon assembly also of the cartridge 4 and of the lid 6, the small arrows represent schematically the flow followed by the oil subjected to detection via the sensor means 20, 21 and to filtering via the cartridge 4.

The flow of lubricating oil is induced, with modalities in themselves known, to come out of by the engine, penetrating into the passage 11 of the base 5. The oil then penetrates into the chamber 3 inside the casing 5-6, ascending it peripherally, and propagating in part up to its top, in the region 3a, but without being able to penetrate into the central channel 4a of the cartridge 4, given the presence of the spacer 15 that functions also as top plug for the channel itself. By virtue of the pressure of the oil entering the chamber 3, the oil itself can then traverse the peripheral wall of the filter cartridge 4. The oil thus purified reaches the central part of the cartridge 4, in which the channel 4a is defined, through which the oil itself is directed towards the conduit 10; from here the purified oil can return into the engine.

Part of the oil introduced into the chamber 3 ascends to the region 3a, impinging upon the sensor means 20, 21, via which the necessary measurements can be made on the oil before this is subjected to purification by the cartridge 4. The areas of passage of the oil concerned, in terms of shapes and sections, (passages 10, 11, channel 4a, meshes of the cartridge 4) are calibrated so that the oil certainly reaches the region 3a of the chamber 3, in order to impinge upon and/or submerge the sensor means 20, 21.

It should be noted that in said configuration, albeit with the sensor means 20, 21 preferably located in a region or chamber 3a not subject to a strong flow or turbulence (i.e., housed in a peripheral chamber or derived from the main path of the passages 10, 11), the oil is in any case continuously replaced in the same region or chamber 3a in order to guarantee a continuous and precise measurement. As has already been said, the sensor means could also comprise a pressure sensor, for example, designed to detect an increase in pressure following upon clogging of the filter cartridge 4.

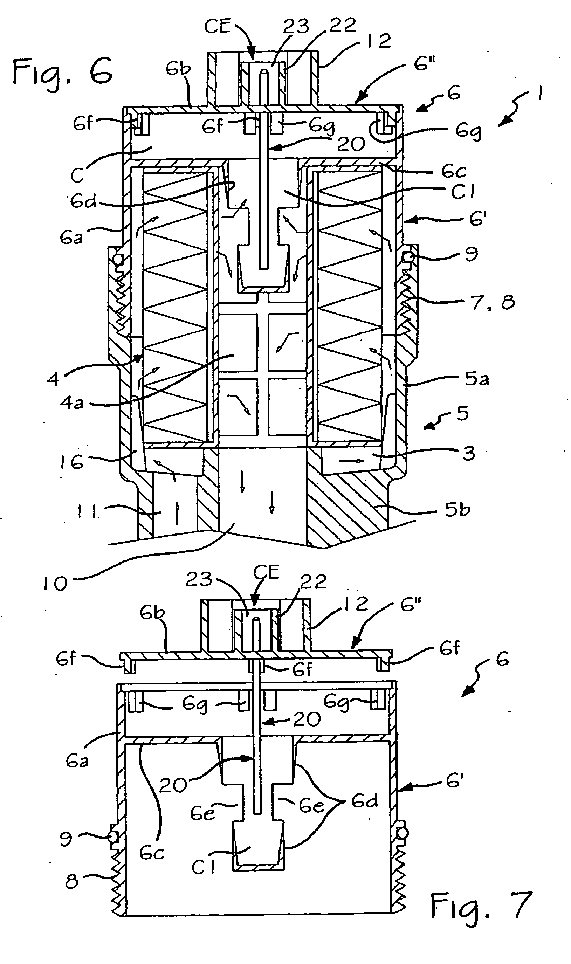

Figures 6 and 7 illustrate an embodiment of the invention, in which the detections are, made by the device on the fluid already filtered. For the purposes of the description of said figures, the same reference numbers as those of the preceding figures are used in order to designate elements that are equivalent to the ones already mentioned.

In this case at least the sensor means 20 are positioned operatively in the central part of the chamber 3 in order to project within the central channel 4a of the cartridge 4, to be in contact with the purified fluid.

As may be seen in Figure 7, in this case the lid 6 is made up of two distinct parts, designated by 6' and 6". The part 6' comprises the cylindrical wall 6a, with the respective thread 8 and gasket 9, as well as an intermediate transverse wall 6c, from which there projects downwards a hollow portion 6d, substantially shaped like a truncated cone, provided in the peripheral wall with slits 6e. The part 6", which is to be fixed in a sealed way at the top to the part 6' (for example, via welding, bonding, screwing, coupling or any other known technique), basically forms the top wall 6b of the lid 6. In this case, the tubular portion 22, which forms part of the connector CE, is formed within the tubular portion 12, on the outside of which the tool for screwing/unscrewing of the lid 6 is to operate. The part 6" is moreover provided with lower projections 6f, designed to co-operate with respective elements in relief 6g obtained in the part 6", in order to provide an anti-rotation system and/or drawing system acting between the two parts, so that, by subjecting the part 6" to an angular movement, the rotation is consequently transferred also to the part 6', in order to screw the lid 6 on the base 5.

The sensor means 20 illustrated in Figures 6 and 7 are conceptually similar to the ones described previously; consequently, also in this case, the connection terminals of the respective electrodes are inserted through respective passages formed in the central area of the wall 6b of the part 6", in a position corresponding to the portion 22, with the respective ends that project into the hollow 23, in a way similar to what was described previously. In said embodiment, the electrodes of the sensor 20 can be substantially rectilinear, with interdigitated (i.e., comblike) teeth.

As may be appreciated from Figure 6, following upon assembly between the parts 6' and 6" of the lid, the part 6" delimits, together with the transverse wall 6c and the cylindrical wall 6a of the part 6', a chamber C, with the electrodes of the sensor 20 that traverse said chamber C and then extend within a chamber C1, delimited by the hollow portion 6d.

In the mounted condition of the filter 1, as represented in Figure 6, the transverse wall 6c of the lid 6 rests on the upper end of the cartridge 4, pressing it downwards to guarantee maintenance of its position and of the respective hydraulic seal, with the hollow portion 6d that projects within the central channel 4a of the cartridge itself.

As in the case of the embodiment illustrated in Figures 1-5, the flow of lubricating oil to be purified is induced to penetrate into the passage 11 of the base 5. The oil then penetrates into the chamber 3 inside the casing 2, ascending it peripherally, at most up to the wall 6c, traversing the filter cartridge 4 and then reaching the channel 4a. Part of the purified oil can in this way enter, via the slits 6e, the chamber C1 delimited by the hollow portion 6d, so as to come into contact with the electrodes of the sensor 20. From the channel 4a the purified oil reaches the conduit 10, and from here can then return into the engine.

It may be noted that, in said conditions, the detection is made on a flow of filtered oil, which fact allows to check the effective filtering efficiency of the cartridge 4.

The configuration referred to in Figures 6 and 7 also enables a detection of the flow in the chamber C1, where dynamic conditions are more present. This is useful particularly in the case where a greater rapidity of detection is necessary and/or it is desired to make a simultaneous detection of the flow also in the chamber C, where less turbulent conditions are, instead, present (the detection in the chamber C can prove useful in the case where there is simultaneously necessary a detection of further parameters, disturbed by said turbulence).

To the sensor 20 illustrated in Figures 6 and 7 there can advantageously be associated one or more of the other sensor means previously mentioned, which could also comprise a pressure sensor, designed to detect a drop in pressure due to clogging of the filter cartridge 4.

In a possible variant, the portion 6d could extend along the periphery of the body 6, instead of at its centre, and have a configuration at least in part arched. In said embodiment, also the electrodes of the sensor 20 could hence have an arched configuration and extend in length in a direction substantially perpendicular to the axis of the device. In another variant, the chamber C could be omitted, with the sensor means 20 that extend entirely just in the chamber C1 (in such an embodiment, the walls 6b and 6c would then be replaced by a single wall).

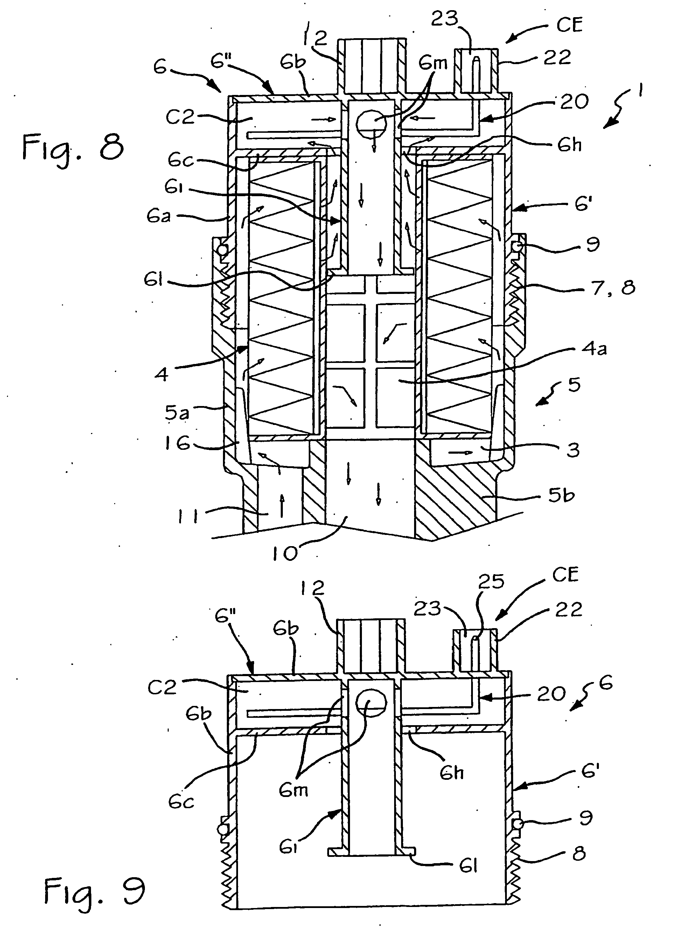

Figures 8 and 9 illustrate a further embodiment of the invention, in which the detections are made by the device 1 on the fluid already filtered. For the purposes of the description of said figures the same reference numbers are used as those of the preceding figures, in order to designate elements that are equivalent to the ones already mentioned.

Also in this variant the lid 6 is made up of two distinct parts 6' and 6", which are designed to be fixed in a sealed way on one another (for example, via welding, bonding, screwing, coupling, or other known technique). In this case, the intermediate transverse wall 6c of the part 6' has a central hole 6h, whilst from the wall 6b of the part 6" there projects downwards a channel 6i. The channel 6i is closed in a position corresponding to the wall 6a, whilst it is open at the lower end, in a position corresponding to which a substantially flange-like peripheral lip 61 projects radially. In the proximity of the upper end of the channel 6i, the same has a series of side holes, some of which are designated by 6m.

As may be appreciated in Figure 9, following upon assembly between the parts 6' and 6" of the lid, the part 6" delimits, together with the transverse wall 6c and the cylindrical wall 6a of the part 6', a chamber C2, in which at least the sensor means 20 are located, the chamber C2 being in any case in communication with the central part or channel 4a of the cartridge 4, as will emerge from what follows.

The sensor means 20 illustrated in Figures 8 and 9 are conceptually similar to the ones described previously, for example, with reference to Figures 4-5, even though, in this case, the respective terminal portions are substantially L-shaped in order to be inserted through respective passages, in a position corresponding to the portion 22, which in this case is formed in a side area of the wall 6b of the part 6".

The length of the channel 6i is such that, in the mounted condition of the device 1, as may be seen in Figure 8, the lower end of the channel itself extends within the central channel 4a of the cartridge 4, with the respective peripheral lip 61 that forms a seal or a restriction on the surface of the cartridge that delimits the channel itself in an intermediate area of the latter. As may be appreciated from Figure 8, moreover, the diameter of the hole 6h of the wall 6c is greater than the diameter of the channel 6i, so that between the two parts there is a passage gap. The holes 6m of the channel are moreover substantially located in a position corresponding to the chamber C2.

In the mounted condition of the filter 1, as represented in Figure 8, the transverse wall 6c of the lid 6 rests on the upper end of the cartridge 4, pressing it downwards to guarantee maintenance of its position and seal, with the channel 6i that projects within the central channel 4a of the cartridge itself. As has been said, the lip 61 operates substantially on the internal surface of the channel 4a, in an intermediate area of the latter.

In operation, the flow of lubricating oil to be purified is induced to penetrate into the passage 11 of the base 5. The oil then penetrates into the chamber 3 inside the casing 2, ascending it peripherally, at the most up to the wall 6c, traversing the filter cartridge 4 and then reaching the channel 4a.

The part of purified oil that reaches the channel 4a below the area in which the lip 61 is operative can traverse the channel itself up to the conduit 10, to return to the engine. On the other hand, the part of purified oil that reaches the channel 4a above the area in which the lip 61 is operative finds outlet at least in part in the gap existing between the hole 6h and the channel 6i, thus reaching the chamber C2 and impinging upon the sensor means 20, 21. The oil can then pass, via the holes 6m, within the channel 6i, and then be conveyed from this into the portion of the channel 4a that extends below the lip 61, and then return to the engine via the conduit 10.

It may be noted that in said configuration, in addition to the already mentioned possibility of measuring a flow of oil already filtered, it is possible to locate the sensor means in a rather spacious chamber C2, even though it is possible to convey on said sensor means a pre-defined and/or optimal part of the flow of oil, calibrated according to the shapes and/or arrangements of the passages 6h, 6i, 6m, etc.

Also in the case of the device illustrated in Figures 8 and 9, there could advantageously be provided one or more of the other sensor means previously mentioned, preferably located in the chamber C2.

In the variant referred to in Figures 8 and 9, to prevent the location of the connector CE from possibly hampering the operations of screwing/unscrewing of the lid 6, the tubular portion 12 can be shaped for receiving within it a tool different from the ones that can be used for the previous embodiments, and in particular a spanner of an Allen type of small size.

Represented in Figures 10 and 11 is a possible variant of the device according to the invention, in accordance with which the sensor means for detecting electrical quantities comprise three electrodes 30, 31 and 32, the electrode 31 being common to the electrodes 30 and 32. Said three electrodes are held in fixed relative, preferably equidistant, positions, without coming into contact with one another, by means of a respective support or frame, which is made of insulating material, for example, overmoulded with thermoplastic material, and is designated by 33 in Figure 10. The frame 33 can comprise coupling elements 33a, designed to couple with respective coupling elements 6r made in the lid or other part of the casing. It may be noted that in Figure 10 the frame 33 is visible in cross-sectional view and the electrodes 30, 31 and 32 are directly in view, whereas in Figure 11 only the electrodes are visible in cross-sectional view.

The electrodes 30 and 32 are built in a way substantially similar to one another and to the electrode 30 illustrated in Figure 5. The electrode 31 is, instead, of conception similar to the electrode 31 illustrated in Figure 5, but with the respective base part 31a that extends according to an arc of a circle of approximately 180°. In this case, moreover, the flat terminal projection 31b of the electrode 31 branches off from one of its teeth 31c set in an intermediate position, instead of from a tooth projecting from one end of the respective base part 31a. As may be appreciated, when the three electrodes 30, 31 and 32 are coplanar, in an operating position within the respective frame 33, the teeth of the common electrode 31 are intercalated with respect to the teeth of the two electrodes 30 and 32, without ever coming into contact therewith, as may be seen in Figure 10. The set of the three electrodes in this case presents an overall shape of a circular sector with a development of approximately 180°, and the sensor comprises in effect two distinct sensors 20a, 20b, which can be controlled independently of one another, for example, one for detecting a value of conductivity (or resistivity) and the other for detecting a value of capacitance.

Represented in Figure 12 is a further possible variant embodiment of the device according to the invention, in which two distinct pairs of electrodes, designated, respectively, by 30, 31 and 32, 34, are schematically represented in cross-sectional view, each pair forming a respective sensor 20a, 20b. Also said electrodes are preferably held in fixed relative position via a respective frame or support (not shown here). The electrodes 30 and 31 illustrated in Figure 12 are substantially configured as the corresponding electrodes illustrated in Figure 5. The electrodes 32 and 34 are of conception similar to the electrodes 30 and 31, but with respective base parts that extend substantially on an arc of a circle of approximately 180°.

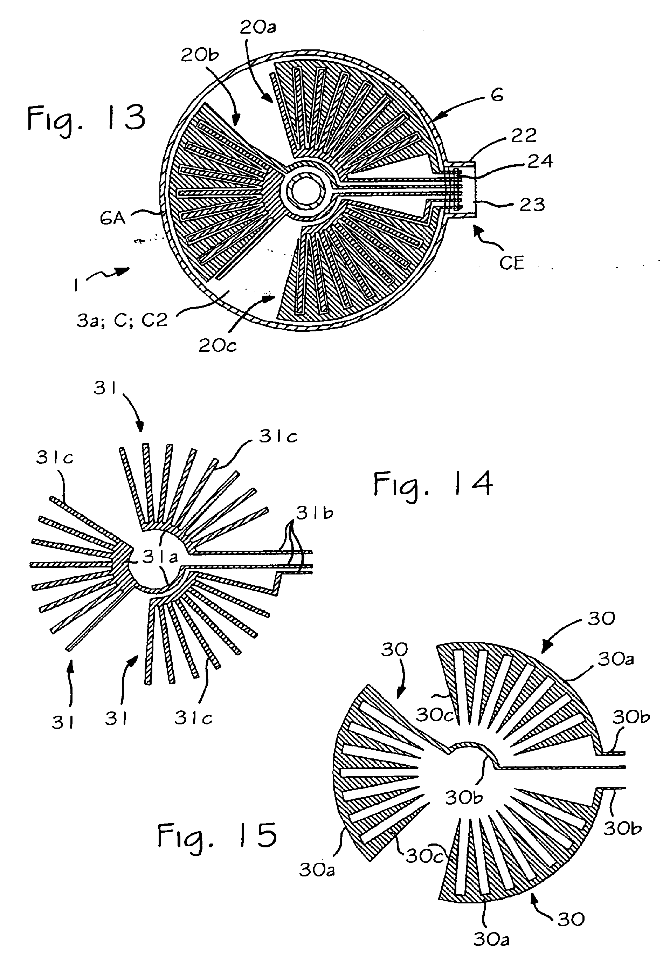

Figure 13 illustrates, instead, the case of use of three distinct pairs of electrodes, which form three distinct sensors 20a, 20b and 20c, evenly distributed and spaced within the part of chamber 3a, or of the chamber C or C2, the various electrodes being preferably supported by a single frame (not represented). As may be seen in Figures 14 and 15, the first and second electrodes of each pair, designated by 30 and 31, respectively, in Figures 15 and 14, are built in a way substantially similar to that of the homologous electrodes illustrated in Figure 5, apart from the respective terminal projection 30b, 31b, which are appropriately shaped so as to reach within the hollow 23 of the connector CE. In this variant, moreover, the base part 30a, 31a of the electrodes 30, 31 of each pair extends according to an arc of a circle of approximately 90° or 100°. In this case the provision of three distinct pairs of electrodes, evenly distributed and spaced within the respective chamber 3a, C or C2, enables availability of three distinct detection sensors 20a, 20b, 20c so that at least one of them is certainly in contact with the oil. The fact of having available three sensors, substantially the same as one another, moreover enables three distinct detections to be performed, on which to be able then to carry out appropriate processing via the electronic control circuit of the device 1, such as, for example, an average of the three values measured, or else a comparison between the three values measured, or else again elimination or deviation of one of the three values detected, etc. Said arrangement likewise enables, if need be, also possible faults to be verified, for example, as a result of a deterioration of one of the electrodes, or else owing to deposits of foreign bodies on the electrodes themselves or between them, etc.

In Figures 10-15, the terminals 30b, 31b of the electrodes are preferably shaped in such a way as to be able to be welded or connected electrically and/or mechanically to a printed circuit 24, for example, with a terminal part of restricted dimensions, said terminal part being preferably provided with an appropriate treatment or coating, designed to facilitate welding or electrical connection.

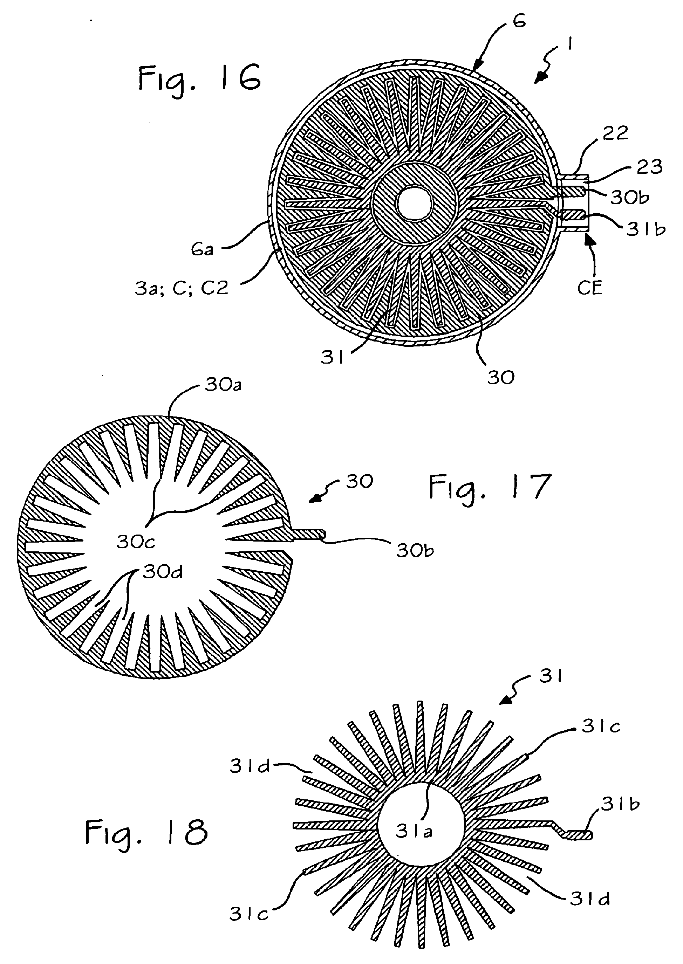

Represented in Figures 16, 17 and 18 is a possible embodiment of the device according to the invention, in which the sensor means 20 are formed by a pair of electrodes 30, 31, which are built in a way conceptually similar to that of the homologous sensors illustrated in Figure 5, but which have a development that is on the whole circular. Moreover visible in Figure 18 is the different shape of the teeth 31c, which have a profile that substantially narrows towards the outer end. Said shape enables having an area of connection that is more ample and provides a greater strength than the base part 31a of the electrode 31, from which the teeth 31c branch off laterally.

As may be noted, the development in length of the circular base part 30a of the electrode 30 is greater than that of the homologous part 31a of the electrode 31 (in other words, the circumference defined by the part 30a is longer than the one defined by the part 31a). The circumference defined by the base part 30a of the electrode 30 is greater than an imaginary circumference touched by the ends or tips of the respective teeth 30c. The circumference defined by the base part 31a of the electrode 31 is, instead, smaller than an imaginary circumference touched by the ends or tips of the respective teeth 31c.

In Figures 16-18 the terminals 30b, 31b of the electrodes are preferably shaped in such a way as to provide at least part of the electrical connector CE, namely, a connector of the type suitable for use in the automotive sector.

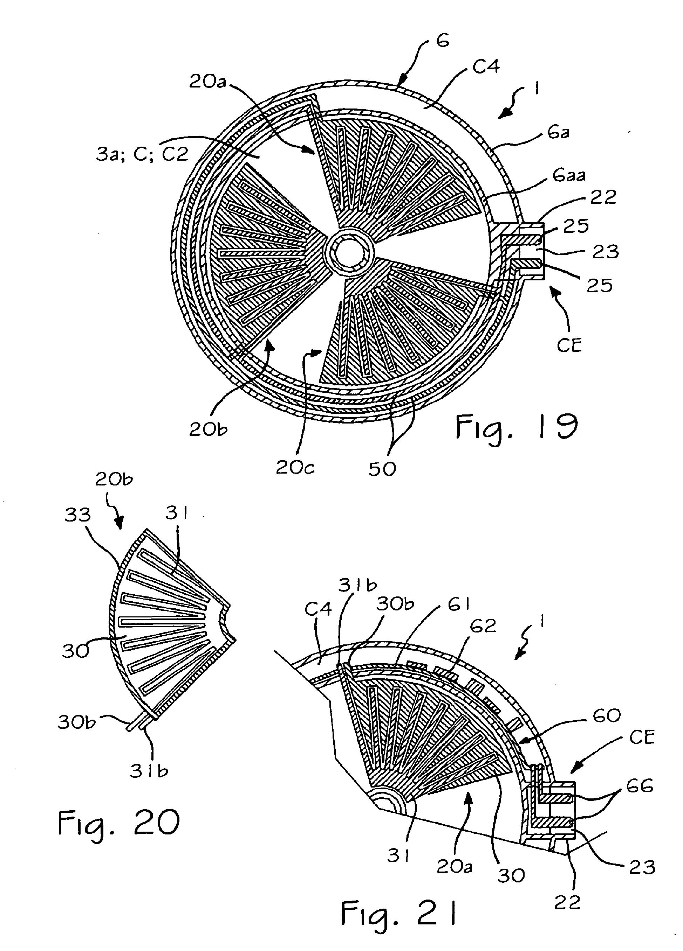

The further variant embodiment illustrated in Figures 19 and 20, is conceptually similar to the one illustrated in Figure 13, envisaging, however, a different shape of the part 6 of the casing 2 and different means of interconnection for the various electrodes 30, 31 of three pairs, i.e., of three sensors 20a, 20b and 20c. Said variant is described, for practical reasons, in relation to the lid 6, but all the corresponding solutions must be considered as enabling integration or association to the base 5 or other part comprised in the casing of the filter, also with shapes and/or arrangements that are more or less different.

In particular, each pair of electrodes 30, 31 has a respective overmoulded frame 33 (visible only in Figure 20), exiting from which is a respective terminal portion 30b, 31b, whilst the lid 6 is configured so as to have an annular peripheral chamber C4, made between two walls 6a and 6aa of the lid 6 and hermetically isolated from the chamber 3a, C or C2 for transit and detection of the fluid. Within the chamber C4 there are at least two connection elements 50, which have a respective end or terminal 50a that projects within the hollow 23 of the connector CE through respective passages appropriately sealed (for example, by means of resin treatment, ovennoulding, etc.). The electrodes of each pair are mounted so that the ends of the terminal portions 30b, 31b that project from the respective frames 33 (see Figure 20) are positioned within the chamber C4, through respective sealed passages in the wall 6aa, for being there connected to the connection elements 50, with modalities in themselves known (welding, electrical spot-welding, etc.). The connection elements 50 could be, for example, in the form of insulated electrical wires or else metal straps spaced at a distance from one another, blanked with appropriate geometries, from sheet metal. The connection elements 50 could advantageously be at least in part moulded directly in the chamber C4, for example, with electrically conductive thermoplastic material and/or overmoulded on the ends or terminals 30b, 31b of the electrodes.

In the chamber C4, which, if need be, could also have a geometry different from the one represented by way of example in Figure 19, there could also be housed an electronic circuit, designed in particular to supervise operation of the sensor means 20, 21. In fact, in Figure 21 possible variant in this sense is represented, in which within the chamber C4 is located an electronic circuit 60, comprising at least one printed circuit 61, for example, of a flexible or appropriately shaped type, on which there are mounted electronic components 62, which comprise, for example, a digital microcontroller and electronic memory means and/or are designed to form at least one measurement circuit and/or data-transmission circuit. In this case, the connection elements 50 and/or the terminal portions 30b, 31b are connected to said printed circuit 61, to which connection terminals 66 for the electrical connector CE are associated.

It may be noted that the electrodes of the sensor means 20 could also not have an arched configuration, but, for example, a rectilinear configuration, according to the positioning region chosen therefor (for example, in the case of the embodiment illustrated in Figure 6-7, the two electrodes can have comb interdigitated teeth).

Irrespective of the various embodiments referred to above, the control circuit of the device according to the invention, whether this is integrated or not in the device itself, preferably comprises at least one measurement circuit, in particular for generating and/or detecting electrical signals, for example, comprising amplification circuits, preferably of a programmable type, at least for the corresponding gain, and/or automatic-calibration circuits, preferably of the type designed to vary a digital datum or the value of a component, such as, for example, a resistor, and/or electronic switches or control or switching devices. Said control circuit preferably comprises also at least one digital processor, such as a microprocessor or a microcontroller, equipped with, or combined to, electronic memory means, preferably of a non-volatile and/or electronically re-writable type, for example, of an EEPROM or Flash type. Said memory means are pre-arranged for storing information in a permanent way even in the absence of electrical supply and/or are designed to be able to be read and written even after being mounted or assembled on the respective electronic circuit. In said memory means, at least part of the control logic and/or of the data necessary for operation of the device 1 is implemented. The aforementioned microcontroller can be supplied through a respective supply stage, of a type in itself know, which can vary the output voltage according to the requirements of the circuit. Moreover a line is preferably associated to the aforesaid circuit, for transmission and/or reception of signals (in the form of logic states 1 and 0, and/or analog data, and/or voltage values, etc.), for example, of a serial type with standard protocol. Said line is preferably used both for programming the microcontroller and for transmitting therefrom information regarding the operation of the circuit 60 and its dialogue with other systems on-board the vehicle (engine control unit, diagnostic systems, etc.).

The aforesaid line can moreover be exploited in the stage of testing of the device. In such a perspective, data detected in the testing stage via the sensor means of the device can be compared with data detected via an external instrument of measurement, such as an oscilloscope or other system for measurement and testing in the production cycle.

During at least one production stage, the electrical and/or hydraulic connection of the device according to the invention to an external testing apparatus can advantageously be envisaged. The device can be connected electrically to the aforesaid electronic apparatus, for example, via the connector CE, and connected hydraulically, via at least one of its conduits for transit of the fluid, to the testing apparatus, which will be designed to supply a specimen of fluid, and thus at least one reference parameter for the test.

A possible phase of testing and/or calibration of the device could then envisage at least one of the following steps:
- detection or measurement of at least one characteristic of said specimen of fluid used as standard via the device 1;
- transmission of the value measured by the device 1 to the electronic testing system;
- comparison of the value measured with a respective standard or reference value;
- processing, according to the aforesaid comparison, of a calibration value, such as, for example, a value of the gain of an amplifier circuit of the device 1; and
- sending of said calibration value to the electronic circuit of the device 1, or, its programming thereof.

The aforesaid steps can, if need be, be repeated a number of times, until the desired measurement value is reached, for example, by varying each time the aforesaid calibration value.

A possible variant may, instead, envisage processing of the values detected directly by the electronic circuit of the device 1. In this case, the phase of testing and/or calibration of the device could then envisage at least one of the following steps:
- detection or measurement of a characteristic of the specimen of fluid used as standard via the device 1;
- transmission of a standard reference value by the electronic testing system to the electronic circuit of the device 1;
- comparison of the value measured with the reference value; and
- processing, according to the aforesaid comparison, of a calibration value, for example, the value of the gain of an amplifier circuit of the device 1.

Also in this variant of the testing and/or calibration cycle, the aforesaid steps can be repeated a number of times until the desired measurement value is reached, finally transmitting an appropriate signal to the automatic control system.

The automatic calibration could envisage writing or recording of at least one datum or value in a memory, preferably of a non-volatile type, and/or the variation of the value of a component, for example, the value of a digital potentiometer, the resistance of which could, for example, be used for definition of the gain of an amplifier circuit, connected to which is at least one of the detection means 20, 21 of the device according to the invention.

The device according to the invention is built in an economically advantageous way, its components being relatively few in number and obtainable conveniently and at a low cost. Also assembly of the device is convenient and fast by virtue of the geometries of the components, which are preferably made in a small number of items and/or with shapes that are complementary to one another.

The basic items of the structure of the device, i.e., the base 5 and the lid 6 can be made of moulded thermoplastic material, in a simple and economically advantageous way. There is nothing in any case to rule out making at least one part of the casing 2 of metal material or else with a series or combination of a number of materials.

Also the sensor means 20 for detecting electrical quantities are obtainable via processes that are in themselves elementary, such as simple operations of blanking for the formation of the electrodes, and simple operations of overmoulding for the formation of the respective frames and/or of the body itself of the device.

It should be emphasized how, according to a further aspect of the invention, the electrodes of the sensor means, which can be used in a device for detecting the characteristics of a fluid, such as in particular a lubricating oil, can be formed by employing operations of moulding or injection-moulding, in particular of a electrically conductive plastic or synthetic material. Said electrodes can be made as separate components and then be mounted on the respective device, or else can be overmoulded on or co-moulded directly with at least part of the casing of the device itself, or together with one or more of its components. Said moulding operations can be, for example, performed employing systems of injection-moulding and/or thermoforming and/or vulcanization of materials, such as thermoplastic and/or thermosetting materials and/or elastomers, and/or other purposely designed equivalent materials suited to the purpose.

In such a solution, the electrodes can be made of substantially insulating thermoplastic material comprising, or containing an electrically conductive material or substance as filler, such as, for example, fibres or powder of carbon, graphite, metallic material, conductive synthetic substances, etc. For said purpose it is particularly advantageous to use a material containing carbon fibre as filler, which is more resistant to chemical and/or electrochemical aggression to the substances in which the electrodes are immersed. In combination or as an alternative, the synthetic material used for forming the electrodes could be of a type intrinsically conductive from the electrical standpoint, by virtue of its molecular structure, without the need for containing fillers or being made with other materials or substances as additives. This is, for example, the behaviour of certain particular, so-called "inherently conductive", polymers. Consequently, where not otherwise specified, and in the annexed claims, the term "electrically conductive synthetic material" is to be understood indifferently as referring to a synthetic material to which there is added a further electrically conductive material or substance, or else a synthetic material intrinsically conductive from the electrical standpoint. Furthermore, the aforesaid synthetic material can also be in the form of electrically conductive ink, appropriately distributed, or silk-screen printed in appropriate regions of the device, or else on one or more of its components or parts thereof.

The electrically conductive synthetic material could also form at least part of an external electrical connector CE, such as its elements of electrical connection. Alternatively, said conductive moulded synthetic material could be electrically connected to metallic terminals of the connector, for example, overmoulded on part of or at one end of said terminals.

In a further variant, the electrodes can envisage an electrically conductive core, subsequently coated or overmoulded with electrically conductive synthetic material, which extends for a substantial part of the length of the electrode. Said core can be formed, for example, by metal material or else by a Carbon fibre having relatively low electrical resistance, made up of a multitude of threads or capillary fibres. Then, an electrically conductive thermoplastic material is overmoulded on said core to form the finished electrodes as distinct elements, which are subsequently assembled on the device, or else as elements directly overmoulded on the device. The material of said core has, in particular, a smaller electrical resistance, or a greater electrical conductivity, as compared to the analogous parameter of said electrically conductive synthetic material.

Consequently, with reference to the device 1 described previously, the electrodes 30, 31 (and/or 32, 34) of the sensor 20 in the various configurations can be formed, instead of by blanked metal strap, via moulding of thermoplastic material, comprising or containing an electrically conductive material as filler, as has just been explained above.

Merely by way of example, the basic thermoplastic material could be PA66 or PPS, with the addition of electrically conductive powder or fibre, in the case of use for the electrodes, and/or as such or with the addition of a glass fibre in the case of use for the body of the device 1 and/or the corresponding connector CE.

In the case where the electrodes 30, 31, 32, 34 are equipped with a supporting frame made of insulating material (for example of the type designated by 33 in Figure 10), this can be co-moulded with, or ovennoulded on, the conductive-plastic electrodes. Another possibility is that of co-moulding the electrodes with, or overmoulding them on, said frame. Said frame could be formed by a part 5, 6 of the casing of the device 1, or else be made during moulding of said part 5, 6, possibly co-moulded with, or overmoulded on, the electrodes 30, 31.

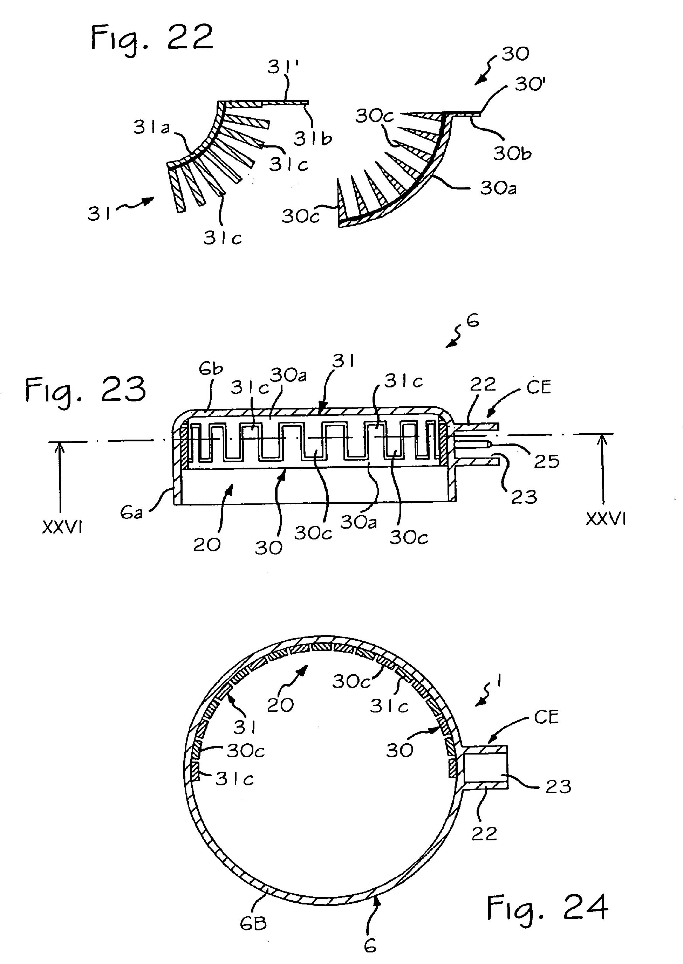

Figure 24 illustrates schematically the case where the electrodes 30, 31 are each equipped with an electrically conductive internal core or an internal core with low electrical resistance, designated by 30', 31', formed, for example, by a chip made of metal material or else with a carbon-fibre thread. On the core 30', 31', which is formed with modalities suited to the purpose, is overmoulded electrically conductive thermoplastic material, to form the finished electrodes 30, 31, equipped with the respective base parts 30a, 31 a, terminal parts 30b, 31 b, and teeth 30c, 31 c.

The electrically conductive thermoplastic materials which can be used for the formation of the electrodes of the device according to the invention typically have a value of electrical resistance of their own. Consequently, the greater the length of the electrode, and/or the smaller the corresponding cross section, the greater the corresponding overall electrical resistance, or rather its different distribution in the different parts of the electrode. For this reason it appears preferable to use, in order to form the electrodes or the connector, conductive synthetic materials with low resistance and/or with a uniform distribution of said value, in particular for the purpose of preventing anomalous distributions of voltages and/or currents in the electrodes, or else in the sensor and/or in the device.

The embodiment illustrated in Figure 22 enables uniform distribution of the current or voltage on the core with low electrical resistance 30', 31', which can have a rough or imprecise shape. In use, the current or voltage is locally distributed by the overlying part made of electrically conductive thermoplastic material, having a more precise shape and arrangement, in so far as it is obtained via moulding, enabling, for example, a uniform and/or precise distance between the surfaces of two facing or opposite electrodes. Said configuration likewise enables a reduction of the negative effect due to the resistance of the conductive mouldable material, which in the case of long paths or electrodes could create different divisions or variations of the electrical signal.

The formation of the electrodes via moulding of electrically conductive synthetic material moreover enables obtaining, in a more convenient and economical way, wider plate surfaces, albeit obtained in the thickness of the respective electrodes, as compared to the case of electrodes formed from metal strap.

Figures 23 and 24 are, instead, schematic illustrations of the case of a sensor 20 overmoulded on a part of the casing of the device 1 according to the invention, and in particular on the lid 6 (it may be noted that in Figure 23 the tubular portion in relief 12 is not represented). In said embodiment, the sensor 20 has a substantially semicircular configuration, with the electrodes 30, 31 overmoulded on the internal peripheral surface of the lid 6 (i.e., on the internal part of its wall 6b), at least in partial relief with respect to the latter. In this case the electrodes 30, 31 have then a substantially arched comb-like shape, with the respective interdigitated teeth. It may be noted that in the case of the present embodiment (as in the one illustrated in Figures 6-7), the teeth 30c, 31c of the electrodes are interdigitated according to a substantially vertical plane. Instead, in the case of the embodiments illustrated in Figures 8-22, the teeth 30c, 31c are interdigitated according to a substantially horizontal axis or an axis in any case transverse with respect to the longitudinal axis of the device 1 or of the cartridge 4.

In order to overmould the electrodes 30c, 31c made of electrically conductive thermoplastic material, a moulding apparatus or mould is used, comprising two parts, of which at least one is mobile, designed to assume, with modalities in themselves known, at least two respective reciprocal positions, i.e., a working, or closing, position, and an opening position. In accordance with the known art, said parts have respective impressions, shaped for defining, as a whole, a main cavity within the die, when they are closed with respect to one another in the working position, it being possible in said main cavity to house the previously obtained body of the component, on which it is desired to overmould the electrodes, for example, the lid 6. In this way, two distinct "differential" cavities are defined in the die, in the space of the main cavity not occupied by the body of the lid 6. Each of said cavities is provided for receiving conductive thermoplastic material designed to form a respective electrode 30, 31, defining its respective external shape. A possible moulding sequence for the purposes of obtaining the electrodes 30, 31 on the lid 6 could comprise, for example, the following steps:
i) insertion of the body of the lid 6 within a first mould part;
ii) closing of the second mould part, whereby the latter is brought substantially to mate with the first mould part, under the thrust of respective forces;
iii) introduction of thermoplastic material, which is intrinsically conductive or else contains appropriate conductive fibres or powder as filler, in an injection-moulding duct of the mould, which is in direct communication with the aforesaid differential cavities, up to filling of the latter;
iv) waiting for enabling cooling and consequent solidification of the conductive thermoplastic material, which thus forms the electrodes 30, 31;
v) opening of the mould parts, which are moved away from one another via respective tensile forces; and
vii) extraction of the lid 6 from one of the mould parts.

Preferably, in order to overmould or co-mould the electrodes 30, 31, in the body of the basic component, for example, the lid 6, appropriate seats, channels or passages are formed, which are to receive anchoring parts of the conductive thermoplastic material forming the overmoulded components, i.e., the electrodes, said seats, channels or passages being operative for withholding the components themselves in position. Said channels could moreover enable or facilitate a further step, for example, comprised between the aforesaid steps (i) and (ii), during which also said core made of electrically conductive material is inserted in the die and/or in said channels.

From the foregoing description there clearly emerge the characteristics of the present invention, as likewise clearly emerge its advantages. The fact that the detection part of the device according to the invention is integrated in a removable part of a casing for a filter cartridge means that the latter is readily insertable in and/or removable from a hydraulic system of a vehicle, without this entailing particular difficulties of assembly/disassembly and/or of operation of the system; i.e., it enables installation of a traditional filter without the detection device, or else insertion of the detection device at a later date, simply by substituting it for a traditional filter, or else replacing a traditional filter casing with the part that integrates the sensor means. The fact that the detecting means will be associated to at least one removable component 5, 6 of the casing of the device and independently of the filtering member, means that the possible replacement of the latter does not involve replacement of the detecting means.

The device is inexpensive to produce as compared to currently available detection sensors and can be readily subjected to periodic maintenance or replacement, in particular during the normal operations of periodic replacement of the filter. For this purpose, the detection device can be readily replaced if need be, or else can be momentarily dismantled for washing in order to restore its proper operation. Said possibility of ease of maintenance also enables the detection device to be produced using technologies and/or materials of lower cost as compared to known sensors, which must necessarily guarantee a longer duration, namely, the entire life of the vehicle without any maintenance. The invention moreover enables prevention of an excessive number of contacts, in particular in the areas inside the device, i.e., in the areas potentially exposed to the fluid in order to prevent a degradation and/or potential failure of said contacts, for example, on account of electrochemical phenomena, or phenomena of oxidation or of deposition.

Of course, without prejudice to the principles of the invention, the details of construction and materials for fabrication thereof, as well as the embodiments, may vary from the ones described and illustrated herein. Furthermore, the individual particulars described herein may be produced or obtained with any other known technique and may in part be omitted, or else, be present in different numbers and with a different arrangement in order to achieve the purposes of the present invention.

The electronic-control circuit integrated in the device according to the invention could be of the type designed to transmit and/or receive data via a wireless communication system to and from a second control circuit located in a remote position, for example, in the passenger compartment of the vehicle, for the purpose of signalling and/or displaying to the driver the state of the fluid and/or of the filter. Said variant presents the advantage of enabling ease of installation of the device according to the invention also on vehicles that were not previously equipped therewith, i.e., on which there is only present a filter mounted directly on the engine.

With reference to the sensor means of the type designated by 20, the electrodes could face the respective plane surfaces of larger size, instead of the respective surfaces of smaller size, defined by the thickness of the original lamina or strap, or else have shapes and/or be made of materials that are completely different, albeit suitable for the purpose. In other variant embodiments, the sensor means 20 could thus comprise electrodes that are substantially the same as one another and are arranged parallel to one another, for example, superimposed on one another, also having a plane, straight or square configuration.

As has already been said, in addition to the detection of intrinsic characteristics of the fluid undergoing analysis, the device according to the invention can advantageously integrate means designed to monitor the operating characteristics of the filter, such as a pressure sensor.

The electrodes of the sensor means 20 could also be built employing other three-dimensional moulding or forming technologies, for example, via sintering or any other technology that may be suitable for producing the electrodes themselves starting from fibre and/or powder of electrically conductive materials.

As has been said, the casing of the device could comprise a number of parts greater than the case illustrated herein, for example, with a component intermediate between the basic component 5 and the end component 6. In this case, at least part of the detecting means 20, 21 could be associated to said intermediate element. Furthermore, at least one of the base parts 5, 6 of the casing of the device according to the invention could be made up of a number of components assembled together. In particular, some parts of the device (such as a connector CE or a module integrating the sensor means) could be made as independent elements, in any case associated to or co-operating with parts of the casing 5, 6 and/or of the device according to the invention, for example, following upon installation of the entire filter or of the device itself in conditions of operation.

The base 5 could comprise internal or external shaped portions, designed to co-operate with tools for assembly and disassembly.

The invention has been described, by way of non-limiting example, with reference to its use in combination with a hydraulic circuit or a filter for a lubricating fluid. It is, however, clear that the invention can be used to advantage in circuits of a different type, and in particular circuits of a vehicle in which a fluid or liquid of a different type is present, such as a fuel, an additive fluid, a conditioning liquid, a washing liquid, a fluid for a braking system, etc.

The device forming the subject of the invention may be produced by associating or combining together at least part of the elements and/or teachings described with reference to the various figures or variants, previously provided merely by way of non-limiting example.

## Claims

1. A filtering device for motor vehicles, comprising means (20, 21) for detecting one or more conditions or characteristics of a fluid flowing in a motor vehicle hydraulic circuit that comprises at least one replaceable filtering member (4) for the fluid, the device having a casing (5-6) delimiting a chamber (3) for removably housing the filtering member (4), the casing (5-6) being formed by a number of components (5, 6) mutually coupled in a separable way, the means for detecting (20, 21) being in contact with the fluid when present in said chamber (3), wherein said detecting means (20, 21) are associated to at least one removable component (5, 6) of the casing (5-6) and in an independent way with respect to said filtering member (4), **characterized in that**
- said detecting means (20, 21) comprise sensor means for detecting an electrical or physical quantity (20), such as a value of conductivity, resistivity, capacitance, impedance, acidity, fluid level, and are pre-arranged for detecting at least one condition of the fluid after transit thereof through the filtering member (4),
- said sensor means for detecting an electrical or physical quantity comprise a sensor (20) including at least two electrodes (30-31; 30-32; 30-32, 34) which have respective surfaces facing one another, preferably substantially equidistant from one another and/or interdigitated with respect to one another, and- said removable component (5, 6) defines at least one first subchamber (C, C1; C2) communicating with said chamber (3) and in which said electrodes (30-31; 30-32; 30-32, 34) are located or extend at least in part.

2. Device according to Claim 1, **characterized in that** said detecting means (20, 21) associated to said removable component (5, 6) also comprise at least one from among:
- temperature-sensing means (21), such as a negative-temperature-coefficient resistor;
- fluidity-sensing means (21), such as a quartz oscillator,
- pressure-sensing means.

3. Device according to Claim 1, **characterized in that** it comprises an electrical connector (CE), in particular electrically connected to at least part of said detecting means (20, 21).

4. Device according to Claim 3, **characterized in that** said connector (CE) is associated to, or forms part of, said removable component (5, 6) and/or is formed at least in part (25; 30c, 31c) with an electrically conductive synthetic material of a mouldable or silk-screen printable type, in particular a thermoplastic material with the addition of electrically conductive material as filler.

5. Device according to Claim 3 or Claim 4, **characterized in that** said electrical connector (CE) comprises at least one from among:
- terminal parts (30b, 31b) of said electrodes (30, 31) and/or
- terminals (25) connected to said detecting means (20) and/or
- terminals (25) connected to a circuit (24), the latter being associated to terminals (30b, 31b) of said electrodes (30, 31) and/or
- sealing means, operative for sealing and/or rendering hermetic at least partially said connector (CE).

6. Device according to Claim 1, **characterized in that** said removable component (5, 6) is an end component of said casing (5-6).

7. Device according to Claim 1, **characterized in that**
- said first subchamber (C, C2) is delimited at least in part at one end (3a) of a space for housing said filtering member (4), and/or
- in said first subchamber (C, C 1; C2) there is located and/or is operative a plurality of said detecting means (20, 21), and/or
- said first subchamber (C, C2) is in fluid communication with a channel (6i) that extends, according to an axial direction of said casing (5-6), towards a central region of said chamber (3), for insertion within a central channel (4a) of said filtering member (4).

8. Device according to Claim 1, **characterized in that** said removable component (5, 6) defines at least one second subchamber (C1).

9. Device according to Claim 8, **characterized in that** said second subchamber (C1)
- communicates both with said chamber (3) and with said first subchamber (C), there extending in said first and second subchambers (C, C1) a respective part of said detecting means (20, 21), and/or
- extends, in an axial direction of said casing (5-6), towards a central region of said chamber (3).

10. Device according to Claim 1, **characterized in that** said removable component (5, 6) comprises a wall (5a, 6a) which is at least in part annular or circular or cylindrical.

11. Device according to Claim 1, **characterized in that** said removable component (6) comprises a peripheral wall (6a), an end wall (6b) and an intermediate wall (6c), which delimit at least part of said first subchamber (C; C2), said intermediate wall (6c) having a passage (6d; 6h) towards said chamber (3).

12. Device according to Claim 11, **characterized in that** from at least one of said end wall (6b) and intermediate wall (6c) there branches off a channel (6d; 6i) that extends, in an axial direction of said casing (5-6), towards a localized region of said chamber (3), where in particular
- said channel (6i) traverses said passage (6h), which is configured as a through hole of said intermediate wall (6c), and/or
- said detecting means (20, 21) are operative within said channel (6d).

13. Device according to Claim 1, **characterized in that** said detecting means (20, 21) comprise at least two sensors (20a, 20b; 20a, 20b, 20c), which have one electrode in common.

14. Device according to Claim 1, **characterized in that** there is provided a support or frame (33) for said electrodes (30-31; 30-32; 30-34, 34) in order to keep them in a fixed position, where in particular said support or frame (33) is overmoulded or moulded jointly with an electrically conductive synthetic material, which preferably forms at least part of said electrodes (30-31; 30-32; 30-32, 34).

15. Device according to Claim 1, **characterized in that** said electrodes (30-31; 30-32; 30-34, 34) have an at least in part arched or circular or curved configuration.

16. Device according to Claim 1, **characterized in that** said electrodes (30-31; 30-32; 30-34, 34) have a mainly rectilinear configuration.

17. Device according to Claim 1, **characterized in that** said electrodes (30-31; 30-32; 30-32, 34) are made of metal and are obtained starting from metal strap, preferably of small thickness.

18. Device according to Claim 1, **characterized in that** said electrodes (30-31; 30-32; 30-32, 34) are formed at least in part with a synthetic material that is electrically conductive or rendered such, where in particular:
- said electrically conductive synthetic material is of a mouldable type, in particular a thermoplastic material with the addition of electrically conductive material as filler, and/or
- said electrically conductive synthetic material is overmoulded on, or distributed or silk-screen printed on, at least part of a body (6) of said removable component (5, 6), or vice versa, or else co-moulded with at least part of said body (6).

19. Device according to Claim 1, **characterized in that** said electrodes (30-31; 30-32; 30-32, 34) have at least one portion (30a, 31a) shaped in conformity with a profile of an internal surface of said removable component (5, 6), in particular with a shape that is at least in part arched or curved.

20. Device according to Claim 18, **characterized in that** said electrodes (30; 31) comprise a core (30'; 31') made of a second electrically conductive material, coated with said electrically conductive synthetic material, where in particular
- said electrically conductive synthetic material is overmoulded on said core (30'; 31'), and/or
- said second electrically conductive material has a smaller electrical resistance or higher conductivity than said electrically conductive synthetic material.

21. Device according to Claim 1, **characterized in that** it comprises an electronic circuit (60).

22. Device according to Claim 21, **characterized in that** said electronic circuit (60) comprises
means for communication, comprising a circuit for wireless communication of signals.

23. Device according to Claim 21, **characterized in that** said electronic circuit (60) is associated to said removable component (5,6).

24. Device according to Claim 21, **characterized in that**, within said casing (5, 6), a further subchamber (C4) is delimited, hermetically isolated from said chamber (3), in which said electronic circuit (60) is housed, where in particular in said subchamber (C4) there are housed at least in part connection means (50) of said detecting means (20, 21) and/or of said electronic circuit (60).

25. Device according to Claim 1, **characterized in that** said removable component (5, 6) comprises at least one shaped-profile portion (12, 13) pre-arranged for co-operating with an assembly/disassembly tool, where in particular said shaped-profile portion (12) has an area or a cavity within which at least part of an electrical connector (CE) is located, preferably connected to at least part of said detecting means (20, 21).

## Patentansprüche

1. Filtervorrichtung für Kraftfahrzeuge, die eine Einrichtung (20, 21) umfasst, mit der ein Zustand oder mehrere Zustände oder Eigenschaft/en eines Fluids erfasst weden, das in einem Kraftfahrzeug-Hydraulikkreis fließt, der wenigstens ein austauschbares Filterelement (4) für das Fluid umfasst, wobei die Vorrichtung ein Gehäuse (5-6) aufweist, das eine Kammer (3) umgrenzt, die das Filterelement (4) entnehmbar aufnimmt, das Gehäuses (5-6) durch eine Anzahl von Einzelteilen (5, 6) gebildet wird, die trennbar miteinander verbunden sind, die Einrichtung (20, 21) zum Erfassen, wenn in der Kammer (3) vorhanden, in Kontakt mit dem Fluid ist und die Erfassungseinrichtung (20, 21) mit wenigstens einem entnehmbaren Einzelteil (5, 6) des Gehäuses (5-6) verbunden und unabhängig von dem Filterelement (4) ist, **dadurch gekennzeichnet, dass**
- die Erfassungseinrichtung (20, 21) eine Sensoreinrichtung umfasst, mit der eine elektrische oder physikalische Größe (20), wie beispielsweise ein Wert der Leitfähigkeit, des Widerstandes, der Kapazität, der Impedanz, des Säuregrades, des Fluidpegels, erfasst wird und sie zum Erfassen wenigstens eines Zustandes des Fluids nach Durchgang desselben durch das Filterelement (4) eingerichtet ist,
- die Sensoreinrichtung zum Erfassen einer elektrischen oder physikalischen Größe einen Sensor (20) umfasst, der wenigstens zwei Elektroden (30-31; 30-32; 30-32, 34) enthält, die jeweilige, einander zugewandte Fläche aufweisen, die vorzugsweise gleichweit voneinander beabstandet sind und/oder ineinander greifen, und das entnehmbare Einzelteil (5, 6) wenigstens eine Teilkammer (C, C1; C2) bildet, die mit der Kammer (3) in Verbindung steht und in der sich die Elektroden (30-31; 30-32; 30-32, 34) befinden oder in die sie sich wenigstens teilweise hinein erstrecken.

2. Vorrichtung nach Anspruch 1, **dadurch gekennzeichnet, dass** die mit dem entnehmbaren Einzelteil (5, 6) verbundene Erfassungseinrichtung (20, 21) des Weiteren umfasst:
- eine Temperatur-Erfassungseinrichtung (21), wie beispielsweise einen NTC-Widerstand (negative-temperature-coefficient resistor);
- eine Fluiditäts-Erfassungseinrichtung (21), wie beispielsweise einen Quarzoszillator, oder/und
- eine Druck-Erfassungseinrichtung.

3. Vorrichtung nach Anspruch 1, **dadurch gekennzeichnet, dass** sie einen elektrischen Verbinder (CE) umfasst, der elektrisch an wenigstens einen Teil der Erfassungseinrichtung (20, 21) angeschlossen ist.

4. Vorrichtung nach Anspruch 3, **dadurch gekennzeichnet, dass** der Verbinder (CE) mit dem entnehmbaren Einzelteil (5, 6) verbunden oder Teil desselben ist und/oder wenigstens teilweise (25; 30c, 31c) mit einem elektrisch leitenden synthetischen Material eines Typs, der geformt oder in Siebdruck verarbeitet werden kann, insbesondere einem thermoplastischen Material, ausgebildet ist, dem elektrisch leitendes Material als Füllstoff zugesetzt ist.

5. Vorrichtung nach Anspruch 3 oder Anspruch 4, **dadurch gekennzeichnet, dass** der elektrische Verbinder (CE) wenigstens eines der folgenden Elemente umfasst
- Anschlussteile (30b, 31b) der Elektroden (30, 31), und/oder
- Anschlüsse (25), die an die Erfassungseinrichtung (20) angeschlossen sind, und/oder
- Anschlüsse (25), die an eine Schaltung (24) angeschlossen sind, wobei letztere mit Anschlüssen (30b, 31b) der Elektroden (30, 31) verbunden sind, und
- eine Dichtungseinrichtung, die in Funktion den Verbinder (CE) abdichtet und/oder wenigstens teilweise luftdicht macht.

6. Vorrichtung nach Anspruch 1, **dadurch gekennzeichnet, dass** das entnehmbare Einzelteil (5, 6) ein Endteil des Gehäuses (5-6) ist.

7. Vorrichtung nach Anspruch 5, **dadurch gekennzeichnet, dass**
- die erste Teilkammer (C, C2) wenigstens teilweise an einem Ende (3a) eines Raums zum Aufnehmen des Filterelementes (4) umgrenzt ist, und/oder
- sich in der ersten Teilkammer (C, C1; C2) eine Vielzahl der Erfassungseinrichtungen (20, 21) befinden und/oder arbeiten, und/oder
- die erste Teilkammer (C, C2) in Fluidverbindung mit einem Kanal (6i) steht, der sich zur Einführung in einen Mittelkanal (4a) des Filterelementes (4) in einer axialen Richtung des Gehäuses (5-6) auf einen Mittelbereich der Kammer (3) zu erstreckt.

8. Vorrichtung nach Anspruch 1, **dadurch gekennzeichnet, dass** das entnehmbare Einzelteil (5, 6) wenigstens eine zweite Teilkammer (C1) bildet.

9. Vorrichtung nach Anspruch 8, **dadurch gekennzeichnet, dass** die zweite Teilkammer (C1)
- sowohl mit der Kammer (3) als auch der ersten Teilkammer (C) in Verbindung steht, wobei sich in der ersten und der zweiten Teilkammer (C, C1) ein jeweiliger Teil der Erfassungseinrichtung (20, 21) erstreckt, und/oder
- sich in einer axialen Richtung des Gehäuses (5-6) auf einen Mittelbereich der Kammer (3) zu erstreckt.

10. Vorrichtung nach Anspruch 1, **dadurch gekennzeichnet, dass** das abnehmbare Einzelteil (5, 6) eine Wand (5a, 6a) umfasst, die wenigstens teilweise ringförmig oder kreisförmig oder zylindrisch ist.

11. Vorrichtung nach Anspruch 1, **dadurch gekennzeichnet, dass** das abnehmbare Einzelteil (6) eine Umfangswand (6a), eine Abschlusswand (6b) und eine Zwischenwand (6c) umfasst, die wenigstens einen Teil der ersten Teilkammer (C; C2) umgrenzen, wobei die Zwischenwand (6c) einen Durchlass (6d; 6h) zu der Kammer (3) hin hat.

12. Vorrichtung nach Anspruch 11, **dadurch gekennzeichnet, dass** von der Abschlusswand (6b) oder/und der Zwischenwand (6c) ein Kanal (6d; 6i) abzweigt, der sich in einer axialen Richtung des Gehäuses (5-6) zu einem örtlich begrenzten Bereich der Kammer (3) hin erstreckt, wobei
- der Kanal (6i) den Durchlass (6h) durchläuft, der als ein Durchgangsloch der Zwischenwand (6c) ausgeführt ist, und/oder
- die Erfassungseinrichtung (20, 21) in dem Kanal (6d) arbeitet.

13. Vorrichtung nach Anspruch 1, **dadurch gekennzeichnet, dass** die Erfassungseinrichtung (20, 21) wenigstens zwei Sensoren (20a, 20b; 20a, 20b, 20c) umfasst, die eine Elektrode gemeinsam haben.

14. Vorrichtung nach Anspruch 1, **dadurch gekennzeichnet, dass** ein Träger oder Rahmen (33) für die Elektroden (30-31; 30-32; 30-34, 34) vorhanden ist, um sie in einer festen Position zu halten, wobei der Rahmen bzw. Träger (33) mit einem elektrisch leitenden synthetischen Material, das vorzugsweise wenigstens einen Teil der Elektroden (30-31; 30-32; 30-34, 34) bildet, umspritzt oder zusammen geformt ist.

15. Vorrichtung nach Anspruch 1, **dadurch gekennzeichnet, dass** die Elektroden (30-31; 30-32; 30-34, 34) wenigstens teilweise bogenförmig oder kreisförmig oder gekrümmt aufgebaut sind.

16. Vorrichtung nach Anspruch 1, **dadurch gekennzeichnet, dass** die Elektroden (30-31; 30-32; 30-34, 34) einen vorwiegend geradlinigen Aufbau haben.

17. Vorrichtung nach Anspruch 1, **dadurch gekennzeichnet, dass** die Elektroden (30-31; 30-32; 30-34, 34) aus Metall bestehen und ausgehend von einem Metallband, vorzugsweise geringer Dicke, hergestellt werden.

18. Vorrichtung nach Anspruch 1, **dadurch gekennzeichnet, dass** die Elektroden (30-31; 30-32; 30-34, 34) wenigstens teilweise mit einem synthetischen Material ausgebildet werden, das elektrisch leitend ist oder gemacht wird, wobei
- das elektrisch leitende synthetische Material ein formbares Material, insbesondere ein thermoplastisches Material, ist, dem elektrisch leitendes Material als Füllstoff zugesetzt ist, und/oder
- das elektrisch leitende Material auf wenigstens einen Teil eines Körpers (6) des abnehmbaren Einzelteils (5) aufgespritzt, verteilt oder im Siebdruck aufgebracht wird, oder umgekehrt, oder ansonsten zusammen mit wenigstens einem Teil des Körpers (6) geformt wird.

19. Vorrichtung nach Anspruch 1, **dadurch gekennzeichnet, dass** die Elektroden (30-31; 30-32; 30-34, 34) wenigstens einen Abschnitt (30a, 31a) haben, der an ein Profil einer Innenfläche des abnehmbaren Einzelteils (5, 6), insbesondere eine Form angepasst geformt ist, die wenigstens teilweise bogenförmig oder gekrümmt ist.

20. Vorrichtung nach Anspruch 18, **dadurch gekennzeichnet, dass** die Elektroden (30; 31) einen Kern (30'; 31') umfassen, der aus einem zweiten elektrisch leitenden Material besteht, das mit dem elektrisch leitenden synthetischen Material überzogen ist, wobei
- das elektrisch leitende synthetische Material auf den Kern (30'; 31') aufgeformt ist, und/oder
- das zweite elektrisch leitende Material einen geringeren elektrischen Widerstand oder höhere Leitfähigkeit hat als das elektrisch leitende synthetische Material.

21. Vorrichtung nach Anspruch 1, **dadurch gekennzeichnet, dass** sie eine elektronische Schaltung (60) umfasst.

22. Vorrichtung nach Anspruch 21, **dadurch gekennzeichnet, dass** die elektronische Schaltung (60) umfasst:
eine Übertragungseinrichtung, die eine Schaltung für drahtlose Übertragung von Signalen umfasst.

23. Vorrichtung nach Anspruch 21, **dadurch gekennzeichnet, dass** die elektronische Schaltung (60) mit dem abnehmbaren Einzelteil (5, 6) verbunden ist.

24. Vorrichtung nach Anspruch 21, **dadurch gekennzeichnet, dass** in dem Gehäuse (5, 6), gegenüber der Kammer (3) hermetisch isoliert, eine erste Teilkammer (C4) umgrenzt ist, und die elektronische Schaltung (60) darin aufgenommen ist, wobei in der Teilkammer (C4) wenigstens teilweise Anschlusseinrichtungen (50) der Erfassungseinrichtung (20, 21) und/oder der elektronischen Schaltung (60) aufgenommen sind.

25. Vorrichtung nach Anspruch 1, **dadurch gekennzeichnet, dass** das abnehmbare Einzelteil (5, 6) wenigstens einen Abschnitt (12, 13) mit geformtem Profil umfasst, der zum Zusammenwirken mit einem Montage-/Demontagewerkzeug eingerichtet ist, wobei der Abschnitt (12) mit geformtem Profil einen Bereich oder einen Hohlraum hat, in dem sich wenigstens ein Teil eines elektrischen Verbinders (CE) befindet, der vorzugsweise an wenigstens einen Teil der Erfassungseinrichtung (20, 21) angeschlossen ist.

## Revendications

1. Dispositif de filtration pour véhicules à moteur, comprenant des moyens (20, 21) pour détecter une ou plusieurs conditions ou caractéristiques d'un fluide s'écoulant dans un circuit hydraulique de véhicule à moteur qui comprend au moins un élément de filtration remplaçable (4) pour le fluide, le dispositif ayant un boîtier (5-6) délimitant une chambre (3) pour loger de manière amovible l'élément de filtration (4), le boîtier (5-6) étant formé par un certain nombre de composants (5, 6) mutuellement couplés d'une manière séparable, les moyens de détection (20, 21) étant en contact avec le fluide lorsqu'il est présent dans ladite chambre (3), dans lequel lesdits moyens de détection (20, 21) sont associés à au moins un composant amovible (5, 6) du boîtier (5-6) et d'une manière indépendante par rapport audit élément de filtration (4), **caractérisé en ce que** :
lesdits moyens de détection (20, 21) comprennent des moyens de capteur pour détecter une quantité électrique ou physique (20), telle qu'une valeur de conductivité, résistivité, capacitance, impédance, acidité, niveau de fluide et sont pré-agencés pour détecter au moins une condition du fluide après son transit par l'élément de filtration (4),
lesdits moyens de capteur pour détecter une quantité électrique ou physique comprennent un capteur (20) comprenant au moins deux électrodes (30-31 ; 30-32 ; 30-32, 34) qui ont des surfaces respectives se faisant face, de préférence sensiblement équidistantes l'une de l'autre et/ou interdigitées l'une par rapport à l'autre, et ledit composant amovible (5, 6) définit au moins une première chambre auxiliaire (C, C1 ; C2) communiquant avec ladite chambre (3) et dans laquelle lesdites électrodes (30-31 ; 30-32 ; 30-32, 34) sont positionnées ou s'étendent au moins en partie.

2. Dispositif selon la revendication 1, **caractérisé en ce que** lesdits moyens de détection (20, 21) associés audit composant amovible (5, 6) comprennent également au moins l'un parmi :
des moyens de détection de température (21), tels qu'une résistance de coefficient de température négative ;
des moyens de détection de fluidité (21), tels qu'un oscillateur à quartz ;
des moyens de détection de pression.

3. Dispositif selon la revendication 1, **caractérisé en ce qu'**il comprend un connecteur électrique (CE), en particulier électriquement raccordé à au moins une partie desdits moyens de détection (20, 21).

4. Dispositif selon la revendication 3, **caractérisé en ce que** ledit connecteur (CE) est associé à, ou fait partie dudit composant amovible (5, 6) et/ou est formé au moins en partie (25 ; 30c, 31c) avec une matière synthétique électriquement conductrice d'un type moulable ou imprimable par sérigraphie, en particulier une matière thermoplastique en ajoutant une matière électriquement conductrice telle qu'une matière de remplissage.

5. Dispositif selon la revendication 3 ou la revendication 4, **caractérisé en ce que** ledit connecteur électrique (CE) comprend au moins l'un parmi :
des parties de borne (30b, 31b) desdites électrodes (30, 31), et/ou
des bornes (25) raccordées auxdits moyens de détection (20), et/ou
des bornes (25) raccordées à un circuit (24), ce dernier étant associé aux bornes (30b, 31b) desdites électrodes (30, 31), et/ou
des moyens d'étanchéité, opérationnels pour rendre étanche et/ou rendre hermétique au moins partiellement ledit connecteur (CE).

6. Dispositif selon la revendication 1, **caractérisé en ce que** ledit composant amovible (5, 6) est un composant d'extrémité dudit boîtier (5-6).

7. Dispositif selon la revendication 1, **caractérisé en ce que** :
ladite première chambre auxiliaire (C, C2) est délimitée au moins en partie au niveau d'une extrémité (3a) d' un espace pour loger ledit élément de filtration (4), et/ou
dans ladite première chambre auxiliaire (C, Cl ; C2), une pluralité desdits éléments de détection (20, 21) est positionnée et/ou est opérationnelle, et/ou
ladite première chambre auxiliaire (C, C2) est en communication de fluide avec un canal (6i) qui s'étend, selon une direction axiale dudit boîtier (5-6), vers une région centrale de ladite chambre (3), pour l'insertion à l'intérieur d'un canal central (4a) dudit élément de filtration (4).

8. Dispositif selon la revendication 1, **caractérisé en ce que** ledit composant amovible (5, 6) définit au moins une deuxième chambre auxiliaire (C1).

9. Dispositif selon la revendication 8, **caractérisé en ce que** ladite deuxième chambre auxiliaire (C1) :
communique à la fois avec ladite chambre (3) et avec ladite première chambre auxiliaire (C), à cet endroit, s'étendant dans lesdites première et deuxième chambres auxiliaires (C, C1), on trouve une partie respective desdits moyens de détection (20, 21), et/ou
s'étend, dans une direction axiale dudit boîtier (5-6), vers une région centrale de ladite chambre (3).

10. Dispositif selon la revendication 1, **caractérisé en ce que** ledit composant amovible (5, 6) comprend une paroi (5a, 6a) qui est au moins en partie annulaire ou circulaire ou cylindrique.

11. Dispositif selon la revendication 1, **caractérisé en ce que** ledit composant amovible (6) comprend une paroi périphérique (6a), une paroi d'extrémité (6b) et une paroi intermédiaire (6c), qui délimitent au moins une partie de ladite première chambre auxiliaire (C ; C2), ladite paroi intermédiaire (6c) ayant un passage (6d ; 6h) vers ladite chambre (3).

12. Dispositif selon la revendication 11, **caractérisé en ce qu'**à partir d'au moins l'une parmi ladite paroi d'extrémité (6b) et une paroi intermédiaire (6c), se ramifie un canal (6d ; 6i) qui s'étend, dans une direction axiale dudit boîtier (5-6), vers une région localisée de ladite chambre (3),
dans lequel en particulier :
ledit canal (6i) traverse ledit passage (6h), qui est configuré comme un trou de passage de ladite paroi intermédiaire (6c), et/ou
lesdits moyens de détection (20, 21) sont opérationnels à l'intérieur dudit canal (6d).

13. Dispositif selon la revendication 1, **caractérisé en ce que** lesdits moyens de détection (20, 21) comprennent au moins deux capteurs (20a, 20b ; 20a, 20b, 20c), qui ont une électrode en commun.

14. Dispositif selon la revendication 1, **caractérisé en ce que** l'on prévoit un support ou châssis (33) pour lesdites électrodes (30-31 ; 30-32 ; 30-34, 34) afin de les maintenir dans une position fixe, dans lequel en particulier ledit support ou châssis (33) est surmoulé ou moulé conjointement avec une matière synthétique électriquement conductrice, qui fait au moins de préférence partie desdites électrodes (30-31 ; 30-32 ; 30-32, 34).

15. Dispositif selon la revendication 1, **caractérisé en ce que** lesdites électrodes (30-31 ; 30-32 ; 30-34, 34) ont au moins en partie, une configuration arquée ou circulaire ou incurvée.

16. Dispositif selon la revendication 1, **caractérisé en ce que** lesdites électrodes (30-31 ; 30-32 ; 30-34, 34) ont une configuration principalement rectiligne.

17. Dispositif selon la revendication 1, **caractérisé en ce que** lesdites électrodes (30-31 ; 30-32 ; 30-32, 34) sont réalisées à partir de métal et sont obtenues à partir d'une bande métallique, de préférence de petite épaisseur.

18. Dispositif selon la revendication 1, **caractérisé en ce que** lesdites électrodes (30-31 ; 30-32 ; 30-32, 34) sont formées au moins en partie avec une matière synthétique qui est électriquement conductrice ou rendue telle, dans lequel, en particulier :
ladite matière synthétique électriquement conductrice est du type moulable, en particulier une matière thermoplastique en ajoutant une matière électriquement conductrice comme une matière de remplissage, et/ou
ladite matière synthétique électriquement conductrice est surmoulée sur, ou distribuée ou sérigraphiée sur au moins une partie d'un corps (6) dudit composant amovible (5, 6), ou vice versa, ou bien co-moulée avec au moins une partie dudit corps (6).

19. Dispositif selon la revendication 1, **caractérisé en ce que** lesdites électrodes (30-31 ; 30-32 ; 30-32, 34) ont au moins une partie (30a, 31a) formée en conformité avec un profil d'une surface interne dudit composant amovible (5, 6), en particulier avec une forme qui est au moins en partie arquée ou incurvée.

20. Dispositif selon la revendication 18, **caractérisé en ce que** lesdites électrodes (30 ; 31) comprennent un noyau (30' ; 31') réalisé avec une deuxième matière électriquement conductrice, recouverte avec ladite matière synthétique électriquement conductrice, dans lequel en particulier :
ladite matière synthétique électriquement conductrice est surmoulée sur ledit noyau (30' ; 31'), et/ou
ladite deuxième matière électriquement conductrice a une plus petite résistance électrique ou une conductivité supérieure à ladite matière synthétique électriquement conductrice.

21. Dispositif selon la revendication 1, **caractérisé en ce qu'**il comprend un circuit électronique (60).

22. Dispositif selon la revendication 21, **caractérisé en ce que** ledit circuit électronique (60) comprend :
des moyens pour la communication comprenant un circuit pour la communication sans fil des signaux.

23. Dispositif selon la revendication 21, **caractérisé en ce que** ledit circuit électronique (60) est associé audit composant amovible (5, 6).

24. Dispositif selon la revendication 21, **caractérisé en ce que**, à l'intérieur dudit boîtier (5, 6), on délimite une autre chambre auxiliaire (C4), hermétiquement isolée de ladite chambre (3), dans laquelle ledit circuit électronique (60) est logé, dans laquelle en particulier, dans ladite chambre auxiliaire (C4), on loge au moins en partie des moyens de raccordement (50) desdits moyens de détection (20, 21) et/ou dudit circuit électronique (60).

25. Dispositif selon la revendication 1, **caractérisé en ce que** ledit composant amovible (5, 6) comprend au moins une partie profilée (12, 13) pré-agencée pour coopérer avec un outil d'assemblage/démontage, dans lequel, en particulier, ladite partie profilée (12) a une zone ou cavité à l'intérieur de laquelle au moins une partie d'un connecteur électrique (CE) est positionnée, de préférence raccordée à au moins une partie desdits moyens de détection (20, 21).
